(19) **Europäisches Patentamt / European Patent Office / Office européen des brevets**

(11) **EP 4 652 490 B1**

(12) **EUROPÄISCHE PATENTSCHRIFT**

(45) Veröffentlichungstag und Bekanntmachung des Hinweises auf die Patenterteilung:
**08.04.2026 Patentblatt 2026/15**

(21) Anmeldenummer: **23838176.8**

(22) Anmeldetag: **29.12.2023**

(51) Internationale Patentklassifikation (IPC):
**G02B 21/22** (2006.01)  **G02B 21/36** (2006.01)
**G02B 21/00** (2006.01)  **G02B 27/28** (2006.01)
**A61B 90/00** (2016.01)

(52) Gemeinsame Patentklassifikation (CPC):
**G02B 21/0092; G02B 21/0012; G02B 21/22; G02B 21/367; G02B 27/283;** A61B 90/20; A61B 2090/371

(86) Internationale Anmeldenummer:
**PCT/EP2023/088028**

(87) Internationale Veröffentlichungsnummer:
**WO 2024/153452 (25.07.2024 Gazette 2024/30)**

(54) **VISUALISIERUNGSANORDNUNG FÜR DIE MIKROCHIRURGIE**

VISUALISATION ASSEMBLY FOR MICROSURGERY

ENSEMBLE DE VISUALISATION POUR MICROCHIRURGIE

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **18.01.2023 DE 102023101104**

(43) Veröffentlichungstag der Anmeldung:
**26.11.2025 Patentblatt 2025/48**

(73) Patentinhaber: **Carl Zeiss Meditec AG 07745 Jena (DE)**

(72) Erfinder:
- **TOTZECK, Michael 73447 Oberkochen (DE)**
- **HAUGER, Christoph 73447 Oberkochen (DE)**

(74) Vertreter: **PATERIS Patentanwälte PartmbB Danckelmannstraße 9 14059 Berlin (DE)**

(56) Entgegenhaltungen:
**WO-A1-2018/047165 US-A1- 2016 091 702**

- **JAEPYEONG CHA ET AL: "Real-time, label-free, intraoperative visualization of peripheral nerves and micro-vasculatures using multimodal optical imaging techniques", BIOMEDICAL OPTICS EXPRESS, vol. 9, no. 3, 12 February 2018 (2018-02-12), United States, pages 1097 - 1110, XP055726529, ISSN: 2156-7085, DOI: 10.1364/ BOE.9.001097**
- **JI QI ET AL: "Mueller polarimetric imaging for surgical and diagnostic applications: a review", JOURNAL OF BIOPHOTONICS, vol. 10, no. 8, 1 August 2017 (2017-08-01), DE, pages 950 - 982, XP055665367, ISSN: 1864-063X, DOI: 10.1002/ jbio.201600152**
- **JÉRÉMY VIZET ET AL: "In vivo imaging of uterine cervix with a Mueller polarimetric colposcope", SCIENTIFIC REPORTS, vol. 7, no. 1, 1 June 2017 (2017-06-01), XP055639295, DOI: 10.1038/ s41598-017-02645-9**

**Beschreibung**

Die vorliegende Erfindung betrifft eine Visualisierungsanordnung für die Mikrochirurgie, zum Beispiel für ein Operationsmikroskop, mit einer Abbildungseinrichtung zum Erzeugen einer Abbildung mit Polarisationskontrast, zum Beispiel einer stereoskopischen Abbildungseinrichtung zum Erzeugen einer stereoskopischen Abbildung mit Polarisationskontrast. Die Erfindung betrifft zudem ein Verfahren zum Erzeugen einer Abbildung mit Polarisationskontrast, zum Beispiel einer stereoskopischen Abbildung mit Polarisationskontrast, eines abzubildenden Objekts mittels einer Visualisierungsanordnung für die Mikrochirurgie. Die Erfindung betrifft darüber hinaus ein Mikroskop, zum Beispiel ein Operationsmikroskop.

Operationsmikroskope werden in verschiedenen Disziplinen der Mikrochirurgie eingesetzt, vorwiegend in der Neuro-, Wirbelsäulen-, HNO- und Augenchirurgie. Sie zeichnen sich vor allem durch die stereoskopische Abbildung mit großen Arbeitsabständen (200mm-600mm) und moderaten Vergrößerungen (bis ca. 20x) aus. Diese Eigenschaften ermöglichen die Verwendung von chirurgischen Instrumenten im sterilen Operations-Feld (OP-Feld) bei gleichzeitiger stereoskopischer Sicht des OP-Situs. In den letzten Jahren ist ein Wechsel von analogen zu digitalen Systemen erfolgt. Bei diesen werden Kameras und 3D-Monitore oder andere 3D-Wiedergabesysteme (Head-Mounted-Displays (HMD), Digitale Okulare (binocular ocular monitors - Boom) für die Bildaufnahme und -darstellung verwendet.

Ein wesentlicher Treiber für die Weiterentwicklung der Operationsmikroskope ist der Wunsch der Anwender nach einer Gewebedifferenzierung. Dies kann je nach Disziplin und Anwendung die Unterscheidung von Tumor und gesundem Gewebe, die Unterscheidung von weißem und grauem Gehirngewebe, die verbesserte Darstellung von Gefäßen und Nerven, die Sichtbarmachung von Phasenobjekten bei Kataraktoperationen (Linse, Kapselsack, etc.) oder die Sichtbarmachung von Membranen bei Eingriffen an der Retina sein. Die digitalen Operationsmikroskope bieten große Vorteile für eine verbesserte Gewebedifferenzierung, weil die verwendeten Kamerasysteme dafür verwendet werden können.

Aus Anwendersicht, insbesondere im Bereich der Neurochirurgie, ergeben sich die folgenden Anforderungen an eine Technologie zur Gewebedifferenzierung. (i) Die Differenzierung der verschiedenen Gewebetypen muss in Echtzeit mit stereoskopischen Bilddaten erfolgen, d.h. ohne merkliche zeitliche Verzögerung für den Anwender, z.B. Chirurgen. Diese Zeit liegt für digitale Systeme im Bereich <50ms. (ii) Die Gewebedifferenzierung muss im gesamten Operationssitus erfolgen, der typischerweise einen Durchmesser von ca. 10mm-50mm hat. Die Auflösung der Gewebedifferenzierung sollte der Auflösung der stereoskopischen Bilddaten entsprechen (analog bzw. digital), sie kann auch geringer sein. (iii) Die Gewebedifferenzierung sollte für den Chirurgen intuitiv interpretierbar und deren applikative Bedeutung durch klinische Studien hinterlegt sein. (iv) Die Gewebedifferenzierung sollte bei Bedarf ein- und ausgeschaltet werden können. (v) Die zur Gewebedifferenzierung verwendete Technologie darf die Kosten und vor allem die Baugröße des Operationsmikroskops nicht wesentlich erhöhen. (vi) Die Gewebedifferenzierung sollte idealerweise ohne die Verwendung von Markern und Farbstoffen erfolgen, da diese eine lange Zulassung erfordern und häufig mit Nebenwirkungen für Patienten verbunden sind.

In der wissenschaftlichen Literatur ist eine große Zahl von Technologien bekannt, die für eine Gewebedifferenzierung verwendet werden können. Dabei haben optische Technologien aufgrund der guten Integrierbarkeit sowie der berührungslosen Messung viele Vorteile gegenüber nicht-optischen Verfahren wie beispielsweise Ultraschall. Die wichtigsten optischen Technologien zur Gewebedifferenzierung sind die Detektion von Fluoreszenz/Autofluoreszenz (kontinuierlich und/oder zeitaufgelöst), Laser-Doppler- und Laser-Speckle-Imaging, Optische Kohärenztomographie (OCT), Raman-Spektroskopie (kohärent oder nichtkohärent), Narrow-Band-Imaging sowie die Detektion der Wirkung von biologischem Gewebe auf den Polarisationszustand von Licht. Der großen Anzahl wissenschaftlicher Publikationen steht eine nur geringe Zahl an in Produkten realisierten Technologien gegenüber, was an den schwer erfüllbaren oben genannten Anforderungen liegt.

In Operationsmikroskopen und auch in Endoskopen haben sich daher in den letzten Jahren vor allem kamerabasierte Fluoreszenztechnologien durchgesetzt, weil sie noch am besten die oben genannten Anforderungen erfüllen. Sie basieren aktuell auf den drei zugelassenen Farbstoffen ICG, NAF und 5-ALA. Sie werden applikativ für eine Differenzierung Tumor/Nicht-Tumor und zur Visualisierung des Blutflusses eingesetzt. Die genannten Fluoreszenztechnologien mit den drei zugelassenen Farbstoffen erfüllt die Anforderungen (i)-(v), nicht jedoch Anforderung (vi), d.h. sie sind nur in Kombination mit einem Medikament einsetzbar. Ein weiterer Nachteil der genannten Fluoreszenzoptionen liegt darin, dass nicht alle applikativ relevanten Gewebetypen unterschieden werden können. Es gibt Tumorarten, die die oben genannten Farbstoffe nicht aufnehmen. Des Weiteren ist es in der Neurochirurgie sehr wichtig Funktionen des Gehirns während des Eingriffs zu schonen. Dazu ist es notwendig, die Faserbahnen des Gehirns (die weiße Substanz) zu erkennen und von Tumoren oder der grauen Substanz des Kortex zu unterscheiden. Dies ist aktuell mit keinem der genannten Farbstoffe möglich. Aus diesem Grund besteht insbesondere in der Neurochirurgie ein großer Bedarf nach einer Technologie zur Gewebedifferenzierung von Tumoren, Gefäßen, sowie der weißen und grauen Substanz.

Von den oben genannten Technologien ist die Polarisation deswegen von Vorteil, weil sie ohne Farbstoffe oder Medikamente auskommt und aufgrund des zugrunde liegenden physikalischen Prozesses sensitiv auf unterschiedliche Gewebetypen reagiert. Tumore sind auf zellulärer Basis von ungeordneter Struktur, während insbesondere Faserbahnen stark geordnete Zonen darstellen. Aus der Literatur ist bekannt, dass sich solche Strukturen unterschiedlich bezüglich ihrer Wirkung auf die Polarisation von eingestrahltem Licht auswirken.

Weitere Mikroskope, in denen Polarisationseigenschaften von Proben untersucht werden, sind aus der US 2016/091702 A1 und aus dem Artikel von Jaepyeong Cha et al., "Real-time, label-free, intraoperative visualization of peripheral nerves and micro-vasculatures using multimodal optical imaging techniques", Biomedical Optics Express, Bd. 9, Nr. 3, 1097-1110 (12.02.2018) bekannt.

Der folgende Abschnitt beschreibt im Detail den Formalismus der Stokes-Vektoren und der Müllermatrix zur Interaktion von optischen Elementen und des Gewebes im OP-Situs mit partiell polarisiertem Licht. Die Müllermatrix ist die 4x4-Transformationsmatrix für den Stokes-Vektor des Beleuchtungslichts, die nach erfolgter Multiplikation den Stokes-Vektor am Ort des Detektors liefern. Die Kenntnis der Müller-Matrix mit ihren 16 Elementen beinhaltet daher alle Informationen, wie das Objekt auf partiell polarisiertes Licht wirkt. Es gibt in der Messtechnik zahlreiche Polarimeter, die die vollständige Müller-Matrix punktuell oder flächig vermessen (z.B. https://mountainphotonics.de/product/axo-axo step/). Ein Müller-Polarimeter besteht aus den Komponenten Lichtquelle, PSG (Polarisation State Generator), PSA (Polarisation State Analyzer) und einem Detektor (punktuell oder flächig). PSG und PSA können dabei auf unterschiedliche Arten realisiert sein, beispielsweise aus rotierenden Verzögerungselementen oder feststehend durch ferroelektrische Elemente. Zum allgemeinen fachlichen Hintergrund wird auf die folgenden Veröffentlichungen verwiesen: W. Singer, M. Totzeck, H. Gross, Handbook of Optical Systems, Vol. 2 "Physical Image Formation", Kap. 26.2.9, S. 475ff in der Reihe "Handbook of Optical Systems", H. Gross (Editor), Wiley VCH [1], H. Engstrom, "Coherency matrix polarization measurements: application to magnetooptic garnet films", Appl. Opt. 30 (1991) 1730-1734 [4], A. Pigula, N. T. Clancy, S. Arya, G. B. Hanna, D. S. Elson in: Video-rate dual polarization multispectralendoscopic imaging, International Society for Optics and Photonics, pp. 93330N-93330N-93334 (2015) [5] und Wei Sheng et al, "Quantitative Analysis of 4 × 4 Mueller Matrix Transformation Parameters for Biomedical Imaging", Photonics 2019, 6, 34; doi:10.3390/photonics6010034 [6].

Im Folgenden wird erläutert, wie aus einem Stokes-Vektor ein Polarisationskontrast, insbesondere für ein zu untersuchendes Gewebe, ermittelt werden kann. Der Stokes-Vektor ist eine in der Polarisationsoptik übliche Methode, um partielle Polarisationszustände darzustellen [1]. Der Stokes-Vektor besteht aus 4 reellen Komponenten, den sog. "Stokesparametern", deren Werte sich aus der Messung der Transmission des Lichtes durch spezifische Polarisatoren ermitteln lassen.

$$\vec{S} = \begin{pmatrix} S_0 \\ S_1 \\ S_2 \\ S_3 \end{pmatrix} = \begin{pmatrix} I_0 \\ P_0 - P_{90} \\ P_{45} - P_{135} \\ P_R - P_L \end{pmatrix} \qquad (1)$$

Dabei steht $I_0$ für die Gesamtintensität, also die Transmission eines neutralen Elementes, $P_0$ für die Transmission durch einen linearen Polarisator unter 0°, $P_{45}$ für die Transmission durch einen linearen Polarisator unter 45°, $P_{90}$ für die Transmission durch einen linearen Polarisator unter 90°, $P_{135}$ für die Transmission durch einen linearen Polarisator unter 135°, $P_R$ für die Transmission durch einen rechtszirkularen Polarisator und $P_L$ für die Transmission durch einen linkszirkularen Polarisator.

Damit stellt der Stokesparameter $S_0$ die Intensität des Lichtes dar. $S_1$ gibt an, wie stark die Differenz zwischen dem in x-Richtung und in y-Richtung linear polarisierten Anteil im Licht ist, also den Anteil der horizontalen bzw. vertikalen linearen Polarisation. $S_2$ gibt an, wie stark die Differenz zwischen dem 45°-und 135°-linear polarisiertem Anteil im Licht ist, also den Anteil der diagonalen linearen Polarisation. $S_4$ gibt an, wie stark die Differenz zwischen dem rechts-und linkszirkular polarisierten Anteil im Licht ist, also den Anteil der zirkularen Polarisation.

Eine wichtige Charakterisierungsgröße von Licht im Gewebekontrast ist der Polarisationsgrad $g$. Das ist der Anteil des polarisierten Lichtes an der Gesamtintensität und errechnet sich aus Formel (1) gemäß:

$$g = \frac{\sqrt{S_1^2 + S_2^2 + S_3^2}}{S_0} \qquad (2)$$

Für polarisierte Beleuchtung reduziert sich in der Regel der Polarisationsgrad durch die Wechselwirkung mit dem Gewebe, unter anderem durch Mehrfachreflexion. Da die Strukturen häufig linear ausgebildet sind (z.B. Nervenfaserbündel), ist insbesondere der lineare Polarisationsgrad $g_{lin}$ relevant

$$g_{lin} = \frac{\sqrt{S_1^2 + S_2^2}}{S_0} \qquad (3)$$

Analog ist der zirkulare Polarisationsgrad $g_{circ}$

$$g_{circ} = \frac{|S_3|}{S_0} \qquad (4)$$

Im Folgenden wird das Prinzip der Messung des Stokes-Vektors erläutert. Obwohl in der Definition des Stokes-Vektors 6 verschiedene Polarisatoren und eine Messung der Gesamtintensität zum Tragen kommen, genügen bereits 4 Polarisatoren, um die Stokesparameter eindeutig zu messen. Das liegt daran, dass jede Summe zweier orthogonaler Polarisationszustände bereits die Gesamtintensität darstellt, also

$$I_0 = P_0 + P_{90} = P_{45} + P_{135} = P_R + P_L \qquad (5)$$

Damit lässt sich der Stokes-Vektor (1) auch wie folgt schreiben

$$\vec{S} = \begin{pmatrix} S_0 \\ S_1 \\ S_2 \\ S_3 \end{pmatrix} = \begin{pmatrix} P_0 + P_{90} \\ P_0 - P_{90} \\ P_{45} - P_0 - P_{90} \\ P_R - P_0 - P_{90} \end{pmatrix} \qquad (6)$$

d.h. durch die Polarisationsmessung von $P_0$, $P_{45}$, $P_{90}$ und $P_R$ ist der Stokes-Vektor eindeutig festgelegt.

Im Folgenden werden die Müllermatrix und ihr Informationsinhalt, insbesondere für Gewebe, erläutert. Die Polarisationswirkung von einem Objekt, wie z.B. Gewebe, besteht darin, einen einfallenden Stokes-Vektor in einen ausfallenden zu transformieren. Im linearen Bereich wird diese Transformation durch Multiplikation mit einer 4x4 Matrix, der Müllermatrix, beschrieben:

$$\vec{S}_{out} = M\vec{S}_{in} \qquad (7)$$

mit der Müllermatrix

$$M = \begin{pmatrix} M_{00} & M_{01} & M_{02} & M_{03} \\ M_{10} & M_{11} & M_{12} & M_{13} \\ M_{20} & M_{21} & M_{22} & M_{23} \\ M_{30} & M_{31} & M_{32} & M_{33} \end{pmatrix} \qquad (8)$$

Die einzelnen Müllermatrix-Elemente haben für sich eine einfache Bedeutung. Sie beschreiben den Anteil der Anregung von einem Stokesparameter durch einen anderen Stokesparameter. So steht z.B. $M_{12}$ für die Anregung von $S_1$ durch $S_2$.

Wie in der Literatur, z.B. in [2], beschrieben, ist für die Polarisationsbeschreibung von Gewebe die Zerlegung der Müllermatrix in elementare Polarisationsmatrizen wichtig. Eine solche Zerlegung ist z.B. die Lu-Chipman polare Zerlegung, in der eine Müllermatrix als Produkt einer Depolarisations-Müllermatrix, einer Retarder-Müllermatrix und einer Diattenuator-Müllermatrix dargestellt wird:

$$M = M_{Dia}M_{Ret}M_{Depol} \qquad (9)$$

mit der Depolarisations-Müllermatrix

$$M_{Depol} = \begin{pmatrix} 1 & 0 & 0 & 0 \\ 0 & a & 0 & 0 \\ 0 & 0 & b & 0 \\ 0 & 0 & 0 & c \end{pmatrix} \qquad (10)$$

der Retarder-Müllermatrix

$$\boldsymbol{M_R} = M_{LR}M_{CR} = \begin{pmatrix} 1 & \vec{0} \\ \vec{0} & \boldsymbol{m_L} \end{pmatrix}\begin{pmatrix} 1 & \vec{0} \\ \vec{0} & \boldsymbol{m_R} \end{pmatrix} \qquad (11)$$

wobei $\vec{0}$ für einen 3-komponentigen 0-Vektor steht und $\boldsymbol{m_L}$ bzw $\boldsymbol{m_R}$ für die 3x3 Submatrizen der linearen und zirkularen Retardance. Die Submatrix des linearen Retarders hängt von 2 Größen ab: dem Betrag der Retardance und der Orientierung, d.h. der Lage einer der beiden Eigenpolarisationen. Der zirkulare Retarder hängt nur vom Betrag der zirkulären Retardance ab. Damit ist $\boldsymbol{M_r}$ durch nur 3 skalare Größen eindeutig beschrieben. Nach [2] ergeben sich die Retardance-Werte aus

$$\delta_L = \cos^{-1}\left(\sqrt{[M_R(1,1) + M_R(2,2)]^2 + [M_R(2,1) + M_R(1,2)]^2}\right) \qquad (12)$$

und

$$\delta_C = tan^{-1}\left(\frac{M_R(2,1) - M_R(1,2)}{M_R(1,1) + M_R(2,2)}\right) \qquad (13)$$

und der Diattenuator-Müllermatrix

$$\boldsymbol{M_D} = \begin{pmatrix} 1 & \vec{D} \\ \vec{D} & \boldsymbol{m_D} \end{pmatrix} \qquad (14)$$

mit dem Diattenuation-Vektor

$$\vec{D} = \frac{1}{M_{00}}[M_{01} \quad M_{02} \quad M_{03}] \qquad (15)$$

und der Submatrix

$$m_D = \sqrt{1 - \left\|\vec{D}\right\|^2}\boldsymbol{I} + \frac{1 - \sqrt{1 - \left\|\vec{D}\right\|^2}}{\left\|\vec{D}\right\|^2}\vec{D}\vec{D}^T \qquad (16)$$

wobei I für die 3x3 Einheitsmatrix steht.

Neben dieser bekannten Zerlegung gibt es in der Literatur der Polarimetrie noch eine ganze Reihe anderer Zerlegungen der Müllermatrix in eine Funktion von Elementarmatrizen [2]. Die Zerlegung liefert eine physikalisch eindeutige und korrekte Darstellung der Müllermatrix aus Depolarisation, Retardance und Diattenuation. Allerdings ist zur korrekten Durchführung der Zerlegung erforderlich, dass auch die gesamte Müllermatrix gemessen wurde. Ist das der Fall, so ist die Müllermatrix-Zerlegung die bevorzugte Analyseform.

In der Regel sind jedoch die Polarisatoren nicht perfekt, sondern müssen selbst wieder durch eine Müllermatrix beschrieben werden. Das kann aber im Messprozess berücksichtigt werden, wie in [4] gezeigt und im Folgenden beschrieben wird. Bereits bei der Messung der Stokes-Vektoren sollte berücksichtigt werden, dass die zur Verfügung stehenden Komponenten polarisationsoptisch nicht ideal sind. Es kann aber angenommen werden, dass sie nicht depolarisieren. Ihre Wirkung auf partiell-polarisiertes Licht wird also durch eine bekannte, aber nicht notwendigerweise einfache Jones-Matrix beschrieben. So könnte z.B. ein Polarisator nur zu einem Polarisationsgrad von 0,9 führen. Statt diese Eigenschaft in das Fehlerbudget mit aufzunehmen ist es sicherlich sinnvoller sie in der Auswertung zu berücksichtigen. Nach [4] kann ein allgemeines Verfahren zur Messung von Stokes-Vektoren (bzw. Polarisationsmatrizen) aufgestellt werden, das nur voraussetzt, dass die Jones-Matrix der polarisationsmodifizierenden Komponenten bekannt ist, aber nicht, dass diese einen bestimmten Wert haben. Basis des Verfahrens ist die Linearität der Polarisationsmatrix-Transformation in der Jones-Matrix

$$\underline{\mathbf{P}}_{out} = \underline{\mathbf{L}}\underline{\mathbf{P}}_{in}\underline{\mathbf{L}}^{+}$$

wobei $P_{in}$ und $P_{out}$ für die Eingangs und Ausgangspolarisationsmatrix und L für die Jones-Matrix des Messsystems stehen. Diese Relation ist linear in $P_{in}$, eine Eigenschaft die auch erhalten bleibt, wenn zur Intensität übergegangen wird

$$I_{out} = \mathrm{Spur}\left(\underline{\mathbf{L}}\underline{\mathbf{P}}_{in}\underline{\mathbf{L}}^{+}\right)$$

Vier Intensitätsmessungen mit vier verschiedenen L gestatten es, ein lineares Gleichungssystem aufzustellen und $P_{in}$ zu bestimmen,

$$I_j = \mathrm{Spur}\left(\underline{\mathbf{L}}_j\underline{\mathbf{P}}_{in}\underline{\mathbf{L}}_j^{+}\right) \quad j = 1,\ldots,4$$

$$= \sum_{k=1}^{4} A_{jk}P_k$$

wobei $P_k$ für die reellen Elemente $P_1$-$P_4$ der Polarisationsmatrix steht. In Matrizen-Schreibweise ergibt sich die Lösung durch Invertierung der Koeffizientenmatrix

$$\vec{I} = \underline{\mathbf{A}}\vec{P} \quad \Rightarrow \quad \vec{P} = \underline{\mathbf{A}}^{-1}\vec{I}$$

Zur Bestimmung der Koeffizienten $A_{jk}$ muss die Gleichung für $I_j$ ausmultipliziert werden.

$$I_j = \mathrm{Spur}\left(\underline{\mathbf{L}}_j\underline{\mathbf{P}}_{in}\underline{\mathbf{L}}_j^{+}\right)$$

$$= \mathrm{Spur}\left\{\begin{pmatrix} L_{xx} & L_{xy} \\ L_{yx} & L_{yy} \end{pmatrix}\begin{pmatrix} P_1 & P_2+iP_3 \\ P_2-iP_3 & P_4 \end{pmatrix}\begin{pmatrix} L_{xx}^{*} & L_{yx}^{*} \\ L_{xy}^{*} & L_{yy}^{*} \end{pmatrix}\right\}$$

$$= \left(\left|L_{xx}\right|^2 + \left|L_{yx}\right|^2\right)P_1 + \left(L_{xx}L_{xy}^{*} + L_{xy}L_{xy}^{*} + L_{yx}L_{yy}^{*} + L_{yy}L_{yx}^{*}\right)P_2$$

$$+ i\left(L_{xx}L_{xy}^{*} - L_{xy}L_{xy}^{*} + L_{yx}L_{yy}^{*} - L_{yy}L_{yx}^{*}\right)P_3 + \left(\left|L_{xy}\right|^2 + \left|L_{yy}\right|^2\right)P_4$$

Damit ergeben sich die Elemente der Koeffizientenmatrix zu

$$A_{j1} = \left|L_{xx}^{j}\right|^2 + \left|L_{yx}^{j}\right|^2$$

$$A_{j2} = 2\,\mathrm{Re}\left\{L_{xx}^{j}L_{xy}^{j*} + L_{yx}^{j}L_{yy}^{j*}\right\}$$

$$A_{j3} = -2\,\mathrm{Im}\left\{L_{xx}^{j}L_{xy}^{j*} + L_{yx}^{j}L_{yy}^{j*}\right\}$$

$$A_{j4} = \left| L^{j}_{xy} \right|^2 + \left| L^{j}_{yy} \right|^2$$

Für die reale Messung müssen die vier verschiedenen Mess-Jones-Matrizen durch polarisationsoptische Komponenten realisiert werden. Dazu gibt es verschiedene Möglichkeiten. Ein Standardverfahren wird in [4] angegeben, wobei ein linearer Polarisator unter 0°, 45° und 90°, sowie ein λ/4 Plättchen mit der langsamen Achse unter 90° gefolgt von einem Polarisator unter 45° verwendet werden. Ein alternatives Messverfahren verwendet ein rotierendes λ/4-Plättchen gefolgt von einem Polarisator. Natürlich sind noch weitere Konfigurationen denkbar. Bei einem "guten" Messverfahren ist nun die Genauigkeit der Messung nicht mehr davon abhängig, wie präzise die Komponenten einen vorgegebenen Wert (z.B. Retardance, Auslöschungsverhältnis) einhalten, sondern nur davon, wie genau dieser Wert bekannt ist. Eine weitere wichtige Größe für die Messgenauigkeit ist die Frage, wie gut die Komponenten eine orthonormale Basis für die Jones-Vektoren aufspannen. Dabei geht der relative Fehler der Stokes-Vektorkomponenten direkt in die Müller-Matrix ein. Bei einer vorgegebenen Genauigkeit der Komponenten wird der relative Fehler aber umso größer sein, je kleiner der vom Messerfahren realisierte zugehörige Basisvektor ist (also die Empfindlichkeit des Messverfahrens für eine gegebene Stokes-Vektor Komponente).

In dem Dokument DE 10 2017 100 904 A1 wird ein Bildwandlungsmodul für ein Mikroskop, welches zur Polarisationsmessung mittels einer Polarisationsmaske ausgelegt ist, beschrieben. In den Dokumenten US 2016/091 702 A1, DE 102 42 983 A1, CN 107490851 A, DE 10 2018 110 806 A1 und WO 2016 170 816 A1 sowie in JAEPYEONG CHA et al: "Real-time, label-free, intraoperative visualization of peripheral nerves and micro-vasculatures using multimodal optical imaging techniques", BIOMEDICAL OPTICS EXPRESS, Bd. 9, Nr. 3, 12. Februar 2018 (2018-02-12), Seiten 1097-1110 werden Operationsmikroskope, welche zur zumindest teilweisen Polarisationsbestimmung ausgelegt sind, beschrieben.

Die größten Herausforderungen für die Integration eines vollständigen Müller-Polarimeters in ein Operationsmikroskop oder allgemein ein Mikroskop, welches Echtzeitauswertungen erfordert, sind die Anforderungen (i) Echtzeitfähigkeit, (ii) Stereoskopie und (v) geringer Bauraum und Kosten (s.o.). Aus diesem Grund wurden in Operationsmikroskopen bisher nur einfache gekreuzte lineare Polarisatoren in Beleuchtung und Beobachtung realisiert (s.u.). In der Literatur sind zahlreiche Ansätze für Polarimeter beschrieben, um die genannten Anforderungen zu erreichen, allerdings bisher ohne Erfolg.

Im ZEISS EXTARO Operationsmikroskop (https://www.zeiss.de/meditec/produkte/zahnheilkunde/operations mikroskope /extaro-300.html) werden ausschließlich lineare Polarisatoren verwendet. Ein erster Polarisator ist im Beleuchtungsstrahlengang des Operationsmikroskops ein- und ausschwenkbar eingebaut, zwei weitere Polarisatoren sind in den beiden Stereostrahlengängen jeweils senkrecht zum Polarisator in der Beleuchtung ebenfalls ein- und ausschwenkbar integriert. Applikativ werden die Polarisatoren zur Unterdrückung von Reflexen auf der Oberfläche von Zähnen eingesetzt ("NoGlare Mode").

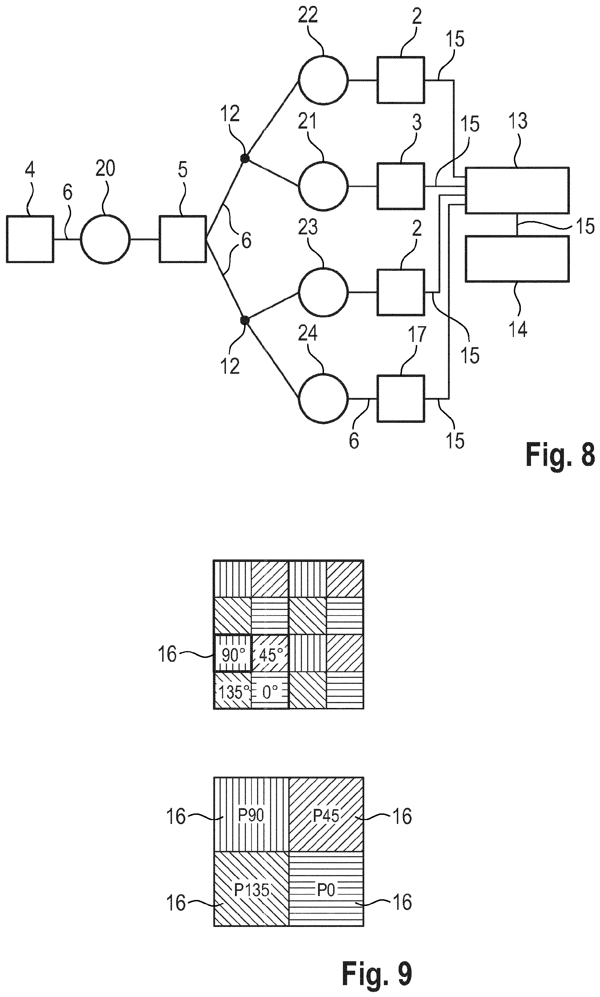

Im "3x3 Müller Polarimetric Endoscope" der Figur 8 der Publikation J. Qi, D. S. Elson, Mueller polarimetric imaging for surgical and diagnostic application, J. Biophotonics10, 950-982 (2017) /DOI 10.1002/jbio.201600152 [2] ist ein linearer Polarisator fest vor dem Beleuchtungsaustritt am distalen Ende des Endoskops positioniert. Das Endoskop wird um die optische Achse rotiert und in drei verschiedenen Positionen während des Messvorgangs fest ausgerichtet. So können in der Beleuchtung drei unterschiedliche Ausrichtungen der linearen Polarisation realisiert werden. Im Beobachtungsstrahlengang vor der Kamera ist ein Filterrad angebracht, in das 3 verschiedene Polarisatoren integriert sind. Mit diesem Endoskop kann eine 3x3 Submatrix der Müllermatrix bestimmt werden. Die Messdauer liegt bei 11,6s. Eine Rotation des Endoskops ist im klinischen Einsatz schwer praktikabel.

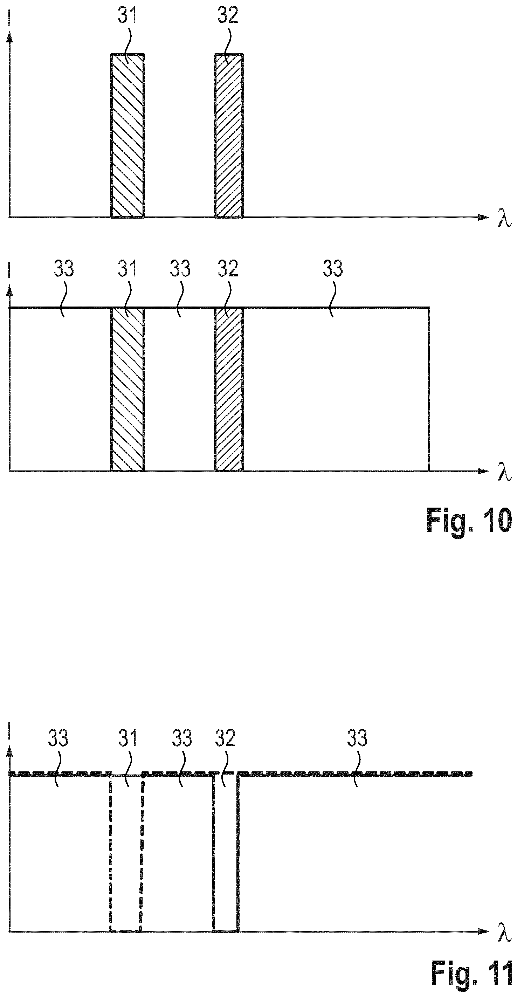

In der Publikation [2] ist ferner ein Endoskop zur Vermessung der kompletten Müller-Matrix mit rotierendem PSG und zeitsequentiellem PSA mit einer Messdauer von 30s beschrieben (siehe Figur 10). Auch dieses Endoskop ist aufgrund der langen Messdauer und der rotierenden Elemente nicht als Produkt für einen klinischen Einsatz oder einen Einsatz, welcher eine Echtzeitauswertung erfordert, geeignet. Die Publikation [2] beschreibt ferner ein Stereoendoskop mit einem linearen Polarisator, der Beleuchtung und einen Stereokanal am distalen Ende abdeckt. Der zweite Stereokanal misst die dazu senkrechte Polarisation (siehe Figur 11). Zusätzlich werden die Polarisationsmessungen mit einem spektralen Narrow-Band-Imaging kombiniert. In diesem Endoskop sind die Anforderungen an die Echtzeitfähigkeit und geringen Bauraum/Kosten erfüllt, es wird jedoch kein stereoskopisches Bild für den Anwender, zum Beispiel einen Chirurgen, erzeugt. Zudem wird nur ein sehr geringer Teil der Müller-Matrix bestimmt (2x2 Submatrix), wesentliche Informationen über die Eigenschaften der Probe werden daher nicht erfasst.

Die Publikation Vizet, J., Rehbinder, J., Deby, S. et al., In vivo imaging of uterine cervix with a Mueller polarimetric colposcope, Sci Rep 7, 2471 (2017), https://doi.org/10.1038/s41598-017-02645-9 [3] beschreibt ein "Müller Polarimetric Colposcope" mit auf ferroelektrischen Modulatoren basierenden PSG und PSA. Das Modul ist unterhalb des Kolposkops befestigt und kann ein- und ausgeschwenkt werden. Die Messdauer liegt bei ca. 1,6 s, vermessen wird die komplette 4x4 Müller-Matrix. Ein Kolposkop entspricht bezüglich des optischen Aufbaus einem Operationsmikroskop. Das Polarimeter-Modul ist in diesem Fall unterhalb des Hauptobjektivs angebracht und besitzt einen monoskopischen Strahlengang. Aus diesem Grund sind bei diesem Ansatz weder die Echtzeitfähigkeit, die Stereoskopie noch eine kompakte Bauweise und geringe Kosten erreicht.

Eine über den einfachen Einsatz gekreuzter Polarisatoren hinausgehende stereoskopische Gewebedifferenzierung in Echtzeit (<50ms) und auf großen Beobachtungsfeldern (>1cm Durchmesser) ist bisher nicht bekannt.

Vor dem beschriebenen Hintergrund ist es Aufgabe der vorliegenden Erfindung, eine vorteilhafte Visualisierungsanordnung für die Mikrochirurgie und ein vorteilhaftes Verfahren zum Erzeugen einer Abbildung mit Polarisationskontrast mittels einer Visualisierungsanordnung für die Mikrochirurgie, sowie ein Mikroskop, insbesondere ein Operationsmikroskops, bereitzustellen.

Die genannten Aufgaben werden durch eine Visualisierungsanordnung für die Mikrochirurgie gemäß der Patentansprüche 1 und 9, ein Verfahren zum Erzeugen einer Abbildung mit Polarisationskontrast mittels einer Visualisierungsanordnung für die Mikrochirurgie gemäß Patentanspruch 10 und ein Mikroskop gemäß Patentanspruch 15 gelöst. Die abhängigen Ansprüche enthalten weitere, vorteilhafte Ausgestaltungen der Erfindung.

Die erfindungsgemäße Visualisierungsanordnung für die Mikrochirurgie, zum Beispiel für ein Operationsmikroskop, umfasst eine Abbildungsvorrichtung, vorzugsweise eine stereoskopische Abbildungsvorrichtung, eine Beleuchtungseinrichtung und eine Polarisationsbestimmungsanordnung. Die Polarisationsbestimmungsanordnung umfasst mindestens zwei Videokameras, welche zum Erfassen von Lichtwellen einer Mehrzahl an Lichtwellenlängen im sichtbaren Wellenlängenbereich, also im Wellenlängenbereich zwischen 400nm und 780nm, ausgelegt sind, und mindestens eine weitere Videokamera, bevorzugt zwei weitere Videokameras, eine Mehrzahl an Polarisationsfiltereinrichtungen und eine Auswertungseinrichtung. Jeder der drei genannten Videokameras ist im Strahlengang ein individueller Teilstrahlengang zugeordnet. Die drei genannten Videokameras können also im Strahlengang nebeneinander oder mit anderen Worten in Bezug auf den Strahlengang parallel zueinander angeordnet sein. Es ist dabei nicht zwingend eine räumlich oder geometrisch parallele Anordnung oder eine räumliche Anordnung nebeneinander gemeint, sondern eine Anordnung, welche die separate Erfassung von Lichtwellen unterschiedlicher Teilstrahlengänge eines Strahlengangs durch die einzelnen Videokameras erlaubt.

Bei den mindestens zwei Videokameras zum Erfassen von mehrfarbigem sichtbarem Licht handelt es sich zum Beispiel um RGB-Kameras. Bei der mindestens einen weiteren Videokamera kann es sich zum Beispiel um eine Fluoreszenz-Kamera handeln.

Es ist mindestens jeweils eine Polarisationsfiltereinrichtung im Strahlengang vor drei der mindestens drei Videokameras, zum Beispiel vor jeder der drei genannten Videokameras, angeordnet. Optional kann mindestens eine Polarisationsfiltereinrichtung im Strahlengang nach der Beleuchtungseinrichtung und vor einem Objektraumbereich, zum Beispiel einem abzubildenden Objekt oder einer Objektebene, angeordnet sein. Die Polarisationsfiltereinrichtungen sind so eingestellt oder einstellbar, dass sich die im Strahlengang vor der mindestens einen genannten weiteren Videokamera und mindestens einer der genannten, zum Erfassen von Lichtwellen einer Mehrzahl an Lichtwellenlängen im sichtbaren Wellenlängenbereich ausgelegten Videokameras angeordneten Polarisationsfilter in ihrer Polarisationswirkung voneinander unterscheiden. Es sind also mindestens zwei, vorzugsweise drei, Polarisationsfiltereinrichtungen vor den Videokameras voneinander abweichend eingestellt oder einstellbar. Zum Beispiel können die Polarisationsfiltereinrichtungen auch so eingestellt oder einstellbar sein, dass sich die im Strahlengang vor den drei genannten Videokameras angeordneten drei Polarisationsfilter in ihrer Polarisationswirkung voneinander unterscheiden, also so, dass jede der drei genannten Videokameras gleichzeitig mit den anderen zwei Videokameras hinsichtlich der Polarisation sich von den anderen Videokameras unterscheidendes Licht empfangen.

Unter einem abzubildenden Objekt wird vorliegend auch ein Subjekt oder ein Teilbereich eines Subjekts, zum Beispiel menschliches oder tierisches oder pflanzliches Gewebe verstanden.

Die Auswertungseinrichtung dazu ausgelegt ist, mittels der von den Videokameras erfassten Bilder Abbildungen, zum Beispiel zwei- oder dreidimensionale Abbildungen, mit Polarisationskontrast zu erzeugen und mittels der Abbildungsvorrichtung anzuzeigen, bevorzugt stereoskopisch anzuzeigen. Hierbei können die erzeugten Abbildungen mit Polarisationskontrast in den Strahlengang der Abbildungsvorrichtung eingeblendet werden. Sie können einer Abbildung ohne Polarisationskontrast teilweise transparent überlagert werden. Es können also die erzeugten Abbildungen mit Polarisationskontrast ein- und ausblendbar sein. Hierbei können die Polarisationsfiltereinrichtungen ein- und ausschaltbar sein.

In einer vorteilhaften Variante ist die Auswertungseinrichtung dazu ausgelegt, die erzeugten Abbildungen mit Polarisationskontrast mittels der Abbildungsvorrichtung mittels mindestens zwei Videokameras, welche zum Erfassen von Lichtwellen einer Mehrzahl an Lichtwellenlängen im sichtbaren Wellenlängenbereich ausgelegt sind, erfassten Weißlicht-Abbildungen überlagert anzuzeigen. Bei den mindestens zwei Videokameras kann es sich um die bereits genannten mindestens zwei Videokameras handeln. Es aber können auch mehr als die drei genannten Videokameras vorhanden sein. Insbesondere kann vor mindestens einer der Videokameras zum Erfassen von mehrfarbigem sichtbarem Licht zumindest sequenziell keine Polarisationsfiltereinrichtung angeordnet sein. Auf diese Weise lassen sich mit mindestens einer der Videokameras zum Erfassen von mehrfarbigem sichtbarem Licht hochaufgelöste mehrfarbige Abbildungen erzeugen, denen die erzeugten Abbildungen mit Polarisationskontrast überlagert werden können. Zum Beispiel kann die Polarisationsfiltereinrichtung vor mindestens einer der Videokameras zum Erfassen von mehrfarbigem sichtbarem Licht so schaltbar sein, dass abwechselnd - bevorzugt in Abständen von weniger als 100ms, insbesondere weniger als 40ms - Abbildungen mit vorgeschaltetem Polarisationsfilter und ohne vorgeschalteten Polarisationsfilter erfasst werden. Dadurch lassen sich hochaufgelöste Abbildungen des abzubildenden Objekts ohne und mit Polarisationskontrast mit derselben Videokamera erfassen. Alternativ dazu kann mindestens eine zusätzliche Videokamera vorhanden sein, wobei mindestens eine Videokamera zum Erfassen einer mehrfarbigen Abbildung des abzubildenden Objekts ohne Polarisationskontrast ausgelegt ist. Der erfassten Abbildung kann eine Abbildung mit Polarisationskontrast überlagert angezeigt werden.

Unter Weißlicht kann zum Beispiel Licht mit einem breitbandigen Farbspektrum verstanden werden. Dabei können die Wellenlängen im Wesentlichen im sichtbaren Bereich liegen. Üblicherweise ist die Verteilung der Wellenlängen kontinuierlich. Für den Menschen entsteht aufgrund des breitbandigen Farbspektrums einer "Weißlichtquelle" ein weißer Farbeindruck. Es geht dabei um den Farbeindruck, der im Auge entsteht. Es handelt sich also um einen physiologischen Effekt und keinen physikalischen Effekt. D.h. ein weißer Farbeindruck kann auf unterschiedlichen Spektren beruhen, die auf den sichtbaren Bereich beschränkt sind.

Die Visualisierungsanordnung kann als Visualisierungsanordnung einer mikrochirurgischen Vorrichtung, zum Beispiel als Visualisierungsanordnung eines Operationsmikroskops, insbesondere eines für neurochirurgische Operationen ausgelegten Operationsmikroskops, ausgestaltet sein. Bevorzugt ist die Visualisierungsanordnung vollständig digital ausgestaltet.

Im Falle einer Ausgestaltung der Visualisierungsanordnung als Komponente eines Mikroskops kann das Mikroskop als vollständig digitales Mikroskop, insbesondere Operationsmikroskop, ausgestaltet sein. Die Visualisierungsanordnung kann dazu ausgelegt sein, Weißlicht-Videodaten und/oder Fluoreszenz-Videodaten stereoskopisch in Echtzeit aufzunehmen und anzuzeigen, insbesondere mit einer erzeugten Abbildungen mit Polarisationskontrast überlagert in Echtzeit anzuzeigen. Im Falle einer Anwendung als Komponente eines Operationsmikroskops kann dieses vorzugsweise für neurochirurgische Operationen ausgelegt sein. Es kann eine Objektfläche oder Objektebene mit Ausdehnung oder einem Durchmesser zwischen 10mm und 50mm aufweisen. Vorzugsweise ist die Auflösung der erzeugten Abbildungen mit Polarisationskontrast niedriger als die Auflösung einer mittels der (stereoskopischen) Abbildungsvorrichtung angezeigten (stereoskopischen) Abbildung ohne Polarisationskontrast oder die Auflösungen entsprechen einander. Zum Beispiel können die genannten Auflösungen weniger als 10 Prozent voneinander abweichen.

In einer weiteren Varianten kann die jeweilige Bedeutung des ermittelten Polarisationskontrasts für die konkrete Anwendung applikativ hinterlegt und in Form einer Anzeige ein- und ausschaltbar sein. Zum Beispiel kann im Falle einer neurochirurgischen Anwendung die Art des polarisationsaufgelöst abgebildeten Gewebes oder aus der polarisationsaufgelösten Abbildung ableitbare Merkmale des Gewebes angezeigt werden. Hierzu kann die komplette Müller-Matrix für einzelne Gewebearten in klinischen Studien bestimmt werden. Aus einer Analyse der Müller-Matrix können für einzelne Gewebearten die klinisch oder allgemein für die jeweilige Anwendung relevanten Elemente abgeleitet werden. Es können dann in Rahmen der Anwendung der Visualisierungsanordnung, z.B. im Rahmen eines Mikroskops, lediglich die relevanten Elemente der Müller-Matrix bestimmt, bzw. die hierfür erforderlichen Stokes-Vektorkomponenten gemessen werden. Die Visualisierungsanordnung kann zur Bestimmung einer festgelegten reduzierten Müller-Matrix ausgelegt sein.

Die erfindungsgemäße Visualisierungsanordnung hat den Vorteil, dass sie eine integrierte Polarisationsmesstechnik bietet, welche die Anforderungen Echtzeitfähigkeit, geringer Bauraum, geringe Kosten sowie optional Stereoskopie erfüllt. In einer Variante als Visualisierungsanordnung eines Operationsmikroskops ermöglicht die vorliegende Erfindung eine verbesserte Gewebedifferenzierung, insbesondere im Bereich der Neurochirurgie. Mittels der erfindungsgemäßen Visualisierungsanordnung lassen sich zeitgleich Daten erfassen, welche das Bestimmen der Stokes-Vektoren und der vollständigen Müllermatrix oder für die jeweilige Anwendung wesentlichen Müllermatrix-Elemente bzw. Müllermatrix-Koeffizienten ermöglichen.

In einer vorteilhaften Variante umfasst mindestens eine der Polarisationsfiltereinrichtungen, zum Beispiel die mindestens drei, z.B. vier, vor den drei, z.B. vier, Videokameras angeordneten Polarisationsfiltereinrichtungen, vorzugsweise alle oben genannten Polarisationsfiltereinrichtungen, eine Mehrzahl sich voneinander unterscheidende Polarisatoren, beispielsweise mindestens vier sich voneinander unterscheidende Polarisatoren. Mindestens eine der Polarisationsfiltereinrichtungen kann ein Filterrad zum Umschalten zwischen einer Mehrzahl an verschiedenen Polarisatoren umfassen. Es kann so jeweils ein Polarisator der Mehrzahl an Polarisatoren in den Strahlengang eingebracht werden. Mindestens eine der Polarisationsfiltereinrichtungen kann zum Beispiel mindestens drei, insbesondere vier, Linearpolarisatoren und optional mindestens einen Zirkularpolarisator umfassen. Hierzu kann die mindestens eine der Polarisationsfiltereinrichtungen ein oder mehrere Verzögerungselemente und/oder mindestens variablen Retarder und/oder ferroelektrische Elemente, etc. umfassen.

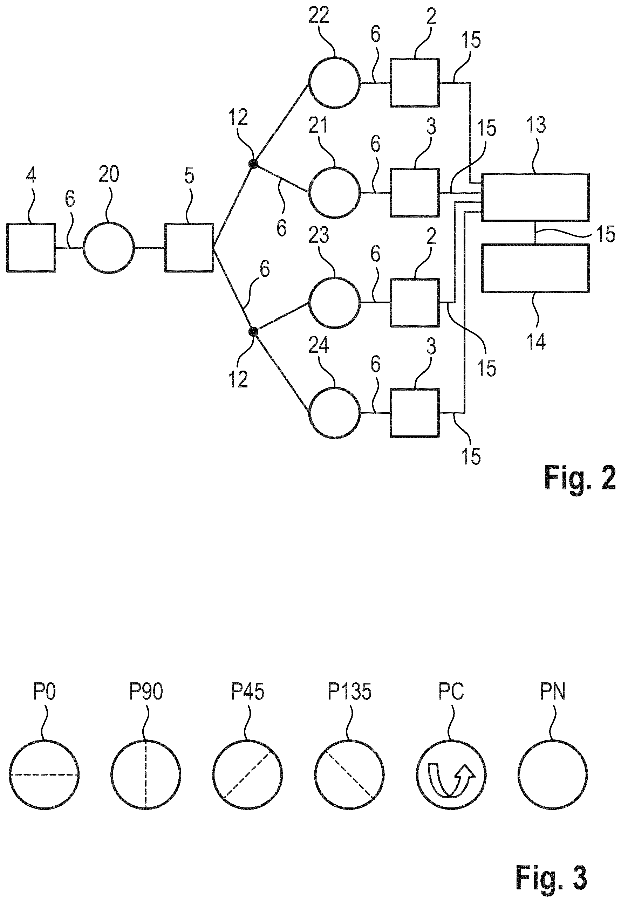

In einer vorteilhaften Einstellung, also bei einer geeigneten Auswahl der in den Strahlengang eingebrachten Polarisatoren der einzelnen Polarisationsfiltereinrichtungen, unterscheiden sich in einer Variante mit vier Kameras von den vier vor den Videokameras angeordneten Polarisatoren mindestens 3 Polarisatoren, vorzugsweise alle 4 Polarisatoren, bezüglich ihrer Polarisationswirkung voneinander. Beispielsweise können 3 der Polarisatoren Linearpolarisatoren, zum Beispiel eine Auswahl aus den in der Figur 3 gezeigten Polarisatoren P0, P45, P90 und P135, und 1 Polarisator ein Zirkularpolarisator sein oder es können 3 der Polarisatoren oder 4 der Polarisatoren Linearpolarisatoren sein. Vor den zum Erfassen von Lichtwellen einer Mehrzahl an Lichtwellenlängen im sichtbaren Wellenlängenbereich ausgelegten Videokameras können hinsichtlich Polarisationswirkung identische Filter angeordnet oder eingestellt werden. Vorzugsweise ist deren Polarisationswirkung orthogonal zu dem optional im nach der Beleuchtungseinrichtung und vor einem abzubildenden Objekt angeordneten Polarisator.

In einer vorteilhaften Variante ist mindestens eine, zum Beispiel zwei, der mindestens einen weiteren Videokamera zum Erfassen von monochromem Licht, also Licht einer definierten Wellenlänge oder eines definierten Wellenlängenbereichs, insbesondere Fluoreszenz-Licht, ausgelegt. Der erfassbare Wellenlängenbereich kann hierbei auch jenseits des sichtbaren Wellenlängenbereichs liegen.

In einer weiteren Variante kann mindestens eine der genannten, z.B. der drei genannten, Videokameras als Polarisationskamera ausgebildet sein. Die Polarisationskamera kann zum Beispiel 4 lineare Polarisatoren auf Pixelebene aufweisen. Die Polarisationskamera kann mindestens einen zirkularen Polarisator aufweisen, zum Beispiel anstelle eines der 4 linearen Polarisatoren, also 3 lineare Polarisatoren und einen zirkularen Polarisator auf Pixelebene. Eine Polarisationsfiltereinrichtung, die vor einer nicht als Polarisationskamera ausgebildeten Videokamera, also vor einer zum Erfassen von Lichtwellen einer Mehrzahl an Lichtwellenlängen im sichtbaren Wellenlängenbereich ausgelegten Videokamera, angeordnet ist, kann einen Zirkularpolarisator umfassen oder als Zirkularpolarisator eingestellt sein. Dies kann in Form einer Kombination aus einem Linearpolarisator und einem Retarder, z.B. Lambda/4-Element unter Null Grad, realisiert sein.

Die Auswertungseinrichtung ist vorteilhafterweise dazu ausgelegt, mittels der von den Videokameras erfassten Bilder einen Satz, insbesondere einen endlichen Satz, von Müllermatrix-Koeffizienten zu bestimmen und einen daraus abgeleiteten Polarisationskontrast zu bestimmen, zum Beispiel zu berechnen.

In einer besonders vorteilhaften Ausgestaltung ist die im Strahlengang nach der Beleuchtungseinrichtung und vor dem Objektraumbereich optional angeordnete Polarisationsfiltereinrichtung dazu ausgestaltet, mindestens zwei festgelegte, voneinander abweichende Wellenlängen, zum Beispiel zwei festgelegte, voneinander abweichende Wellenlängenbereiche, gleichzeitig voneinander abweichend, also unterschiedlich, zu polarisieren. Dies ermöglicht eine zeitgleiche Erfassung einer größeren Anzahl an Daten, mit welchen wiederum eine größere Anzahl an Müllermatrix-Koeffizienten bestimmt bzw. ermittelt werden kann. Hierdurch lassen sich stärkere Polarisationskontraste bestimmen und somit qualitativ hochwertige Abbildungen mit Polarisationskontrast erzeugen. Zudem können die simultan zur Verfügung stehenden Detektionskanäle (z. B. die Anzahl der Kamerasensoren bei RGB-Kameras), gegebenenfalls spektral kodierte Detektionskanäle, für unterschiedliche Polarisationsinformationen eines zu untersuchenden Objekts genutzt werden. Die Polarisationen und Spektren können also so ausgelegt sein, dass eine Mehrzahl an Detektionskanälen, z.B. jeder Detektionskanal, unterschiedliche Informationen, z.B. Gewebeinformationen, liefert.

Mindestens eine der Polarisationsfiltereinrichtungen kann einen Farbfilter oder Wellenlängenfilter und/oder einen wellenlängenselektiven bzw. wellenlängenspezifischen Polarisator umfasst. Hiermit kann insbesondere die zuvor beschriebene Ausgestaltung realisiert werden. Die jeweilige Polarisationsfiltereinrichtung kann zum Beispiel einen Notch-Filter, insbesondere einen polarisierenden Notch-Filter, und/oder einen Bandpassfilter, insbesondere einen polarisierenden Bandpassfilter, umfassen.

Allgemein können die genannten Polarisationsfiltereinrichtungen Filter für Fluoreszenz und/oder Polarisation, insbesondere wellenlängenspezifische Polarisation, umfassen. Zum Beispiel können ein erster Polarisationszustand für mindestens eine erste Wellenlänge oder einen ersten Wellenlängenbereich und ein zweiter Polarisationszustand für mindestens eine zweite Wellenlänge oder einen zweiten Wellenlängenbereich eingestellt oder einstellbar sein.

Die Beleuchtungseinrichtung kann zur Abstrahlung von bichromatischem Licht oder polychromatischem Licht ausgelegt sein. Vorzugsweise ist die Beleuchtungseinrichtung zur Abstrahlung von polychromatischem Licht ausgelegt und die im Strahlengang nach der Beleuchtungseinrichtung und vor dem Objektraumbereich angeordnete Polarisationsfiltereinrichtung umfasst mindestens einen, zum Beispiel zwei, Notch-Filter, bevorzugt polarisierenden Notch-Filter, und/oder mindestens einen, zum Beispiel zwei, Bandpassfilter, bevorzugt polarisierenden Bandpassfilter.

Weiterhin kann die Visualisierungsanordnung mindestens einen Strahlteiler, zum Beispiel ein dichroitischer Strahlteiler, aufweisen. Dieser kann im Strahlengang zwischen dem Objektraumbereich und den Videokameras angeordnet sein.

Mindestens eine der Videokameras, welche zum Erfassen oder Empfangen von Lichtwellen einer Mehrzahl an Lichtwellenlängen im sichtbaren Wellenlängenbereich ausgelegt ist, kann als 3-Chip-Kamera oder als 1-Chip-Kamera ausgestaltet sein.

Eine zu der zuvor beschriebenen Visualisierungsanordnung alternative erfindungsgemäße Visualisierungsanordnung für die Mikrochirurgie umfasst eine Abbildungsvorrichtung, z. B. eine stereoskopische Abbildungsvorrichtung, eine Beleuchtungseinrichtung und eine Polarisationsbestimmungsanordnung. Die Polarisationsbestimmungsanordnung umfasst zwei Polarisationskameras, wobei jeder der zwei Polarisationskameras im Strahlengang ein individueller Teilstrahlengang zugeordnet ist, und eine Auswertungseinrichtung. Die Auswertungseinrichtung ist dazu ausgelegt, mittels der von den Polarisationskameras erfassten Bilder Abbildungen mit Polarisationskontrast zu erzeugen und mittels der Abbildungsvorrichtung anzuzeigen, vorzugsweise stereoskopisch anzuzeigen. Die alternative erfindungsgemäße Visualisierungsanordnung bietet eine zu der oben beschriebenen erfindungsgemäßen Visualisierungsanordnung gleichwertige Lösung. Beide Varianten nutzen dieselben technischen Effekte zum Erzeugen von Abbildungen mit Polarisationskontrast und haben dieselben - oben ausführlich beschriebenen - Merkmale und Vorteile. Insbesondere kann die alternative erfindungsgemäße Visualisierungsanordnung die oben im Zusammenhang mit der zuerst beschriebenen erfindungsgemäßen Visualisierungsanordnung optionalen Merkmale und Eigenschaften aufweisen.

Die Auswertungseinrichtung ist dazu ausgelegt, die erzeugten Abbildungen mit Polarisationskontrast Weißlicht-Abbildungen überlagert anzuzeigen, wobei die Weißlicht-Abbildungen mittels der zwei Polarisationskameras erfasst werden. Die Anzeige erfolgt dabei mittels der Abbildungsvorrichtung.

Das erfindungsgemäße Verfahren zum Erzeugen einer Abbildung mit Polarisationskontrast, z.B. einer stereoskopischen Abbildung mit Polarisationskontrast, eines Objekts mittels einer zuvor beschriebenen erfindungsgemäßen Visualisierungsanordnung für die Mikrochirurgie, insbesondere für ein Operationsmikroskop, umfasst folgende Schritte: Mittels der Beleuchtungseinrichtung werden Lichtwellen, insbesondere im sichtbaren Wellenlängenbereich und optional darüber hinaus, auf das Objekt eingestrahlt. Mittels der mindestens zwei Polarisationskameras oder mindestens drei Videokameras werden Lichtwellen, welche nach einer Wechselwirkung des Objekts mit den eingestrahlten Lichtwellen von dem Objekt abgestrahlt werden, erfasst oder empfangen. Es wird eine Abbildung mit Polarisationskontrast, bevorzugt eine stereoskopischen Abbildung mit Polarisationskontrast, basierend auf einem generierten Polarisationszustand der eingestrahlten Lichtwellen und/oder einem analysierten Polarisationszustand der erfassten oder empfangenen Lichtwellen erzeugt. Das erfindungsgemäße Verfahren hat die bereits beschriebenen Merkmale und Vorteile der erfindungsgemäßen Visualisierungsanordnung.

In einer vorteilhaften Variante werden Lichtwellen eines definierten Polarisationszustandes mittels der Beleuchtungseinrichtung und einer im Strahlengang nach der Beleuchtungseinrichtung und vor dem Objekt angeordneten Polarisationsfiltereinrichtung eingestrahlt. Der Polarisationszustand der empfangenen Lichtwellen kann mittels der genannten Videokameras und der im Strahlengang vor diesen angeordneten Polarisationsfiltereinrichtungen analysiert werden. Mittels der Auswertungseinrichtung kann eine Abbildung, z.B. eine stereoskopischen Abbildung, mit Polarisationskontrast basierend auf einem analysierten Polarisationszustand der erfassten oder empfangenen Lichtwellen erzeugt werden, wobei mittels der von den Videokameras und/oder den Polarisationskameras erfassten Bilder ein Satz von Müllermatrix-Koeffizienten bestimmt wird und ein daraus abgeleiteter Polarisationskontrast bestimmt, insbesondere berechnet, wird.

Die Abbildung, z.B. stereoskopische Abbildung, mit Polarisationskontrast wird vorzugsweise innerhalb eines Zeitintervalls von weniger als 50ms erzeugt. Die Auflösung der erzeugten Abbildung, z.B. stereoskopischen Abbildung, mit Polarisationskontrast weicht vorzugsweise weniger als 10 Prozent von der Auflösung einer entsprechenden Abbildung, z.B. stereoskopischen Abbildung, ohne Polarisationskontrast ab. Besonders bevorzugt wird Abbildung, z.B. die stereoskopische Abbildung, mit Polarisationskontrast innerhalb eines Zeitintervalls von weniger als 50ms erzeugt und einer mehrfarbigen Abbildung, z.B. einer stereoskopischen Abbildung, überlagert angezeigt.

Im Rahmen des erfindungsgemäßen Verfahrens kann die Photogrammetrie auf die Videosignale angewendet werden, um für die polarimetrische Auswertung jeweils korrespondierende Pixel, z.B. in den Stereokanälen, zu identifizieren. Dabei kann pixelweise ein Zahlenwert berechnet werden, der eine Polarisationseigenschaft wiederspiegelt. Daraus kann pixelweise ein Farbwert abgeleitet werden, der die Polarisationseigenschaft wiederspiegelt. Es kann eine insbesondere stereoskopische Darstellung der Polarisationseigenschaft alleine oder dem Weißlichtbild überlagert in Echtzeit erfolgen.

Das erfindungsgemäße Mikroskop, bei welchem es sich um ein Operationsmikroskop - zum Beispiel neurochirurgisches Operationsmikroskop - handeln kann, umfasst eine oben beschriebene erfindungsgemäße Visualisierungsanordnung und/oder ist dazu ausgelegt, ein zuvor beschriebenes erfindungsgemäßes Verfahren auszuführen. Es hat die oben bereits genannten Merkmale und Vorteile. Das erfindungsgemäße Mikroskop ist bevorzugt stereoskopisch und/oder teilweise oder vollständig digital ausgestaltet.

Das Mikroskop, insbesondere das Operationsmikroskop, kann eine Optik mit variabler Brennweite, also eine Optik mit variablem Fokus (Varioskop), und/oder mindestens ein Objektiv, welches im Strahlengang vor mindestens einer der Videokameras angeordnet ist, und/oder mindestens eine Zoom-Optik und/oder eine Umfeldkamera, also eine Videokamera zum Erfassen eines Raumbereichs um einen mikroskopisch abzubildenden Objektbereich herum, umfassen.

Die Erfindung wird im Folgenden anhand von Ausführungsbeispielen unter Bezugnahme auf die beigefügten Figuren näher erläutert. Obwohl die Erfindung im Detail durch die bevorzugten Ausführungsbeispiele näher illustriert und beschrieben wird, so ist die Erfindung nicht durch die offenbarten Beispiele eingeschränkt und andere Variationen können vom Fachmann hieraus abgeleitet werden, ohne den Schutzumfang der Erfindung zu verlassen.

Die Figuren sind nicht notwendigerweise detailgetreu und maßstabsgetreu und können vergrößert oder verkleinert dargestellt sein, um einen besseren Überblick zu bieten. Daher sind hier offenbarte funktionale Einzelheiten nicht einschränkend zu verstehen, sondern lediglich als anschauliche Grundlage, die dem Fachmann auf diesem Gebiet der Technik Anleitung bietet, um die vorliegende Erfindung auf vielfältige Weise einzusetzen.

Der hier verwendete Ausdruck "und/oder", wenn er in einer Reihe von zwei oder mehreren Elementen benutzt wird, bedeutet, dass jedes der aufgeführten Elemente alleine verwendet werden kann, oder es kann jede Kombination von zwei oder mehr der aufgeführten Elemente verwendet werden. Wird beispielsweise eine Zusammensetzung beschrieben, die die Komponenten A, B und/oder C, enthält, kann die Zusammensetzung A alleine; B alleine; C alleine; A und B in Kombination; A und C in Kombination; B und C in Kombination; oder A, B, und C in Kombination enthalten.

Fig. 1     zeigt schematisch ein erfindungsgemäßes Operationsmikroskop mit einer erfindungsgemäßen Visualisierungsanordnung in Form eines Blockdiagramms.

Fig. 2     zeigt schematisch in Form eines Blockdiagramms die polarisationsoptische Wirkungsweise einer erfindungsgemäßen Visualisierungsanordnung.

Fig. 3     zeigt schematisch beispielhafte Polarisationsfilter.

Fig. 4     zeigt schematisch in Form eines Blockdiagramms die polarisationsoptische Wirkungsweise einer beispielhaften Variante einer erfindungsgemäßen Visualisierungsanordnung.

Fig. 5     zeigt schematisch in Form eines Blockdiagramms die polarisationsoptische Wirkungsweise einer beispielhaften Variante einer erfindungsgemäßen Visualisierungsanordnung.

Fig. 6     zeigt schematisch in Form eines Blockdiagramms die polarisationsoptische Wirkungsweise einer beispielhaften Variante einer erfindungsgemäßen Visualisierungsanordnung.

Fig. 7     zeigt schematisch in Form eines Blockdiagramms die polarisationsoptische Wirkungsweise einer beispielhaften Variante einer erfindungsgemäßen Visualisierungsanordnung.

Fig. 8     zeigt schematisch in Form eines Blockdiagramms die polarisationsoptische Wirkungsweise einer beispielhaften Variante einer erfindungsgemäßen Visualisierungsanordnung.

Fig. 9     zeigt schematisch das Prinzip einer Polarisationskamera.

Fig. 10     zeigt schematisch zwei Diagramme, welche mögliche Beleuchtungen des Objektbereichs darstellen.

Fig. 11     zeigt schematisch ein Diagramm, welches die Realisierung einer in der Figur 10 unten gezeigten Beleuchtung mittels polarisierender Notch-Filter veranschaulicht.

Fig. 12     zeigt schematisch eine Realisierung der in der Figur 11 gezeigten Variante mittels polarisierender Bandpass-Filter.

Fig. 13     zeigt schematisch in Form eines Blockdiagramms die polarisationsoptische Wirkungsweise einer beispielhaften Variante eines erfindungsgemäßen Visualisierungsanordnung unter Verwendung von zwei verschiedenen Wellenlängen.

Fig. 14     zeigt schematisch in Form eines Blockdiagramms die polarisationsoptische Wirkungsweise einer beispielhaften Variante einer erfindungsgemäßen Visualisierungsanordnung unter Verwendung von zwei verschiedenen Wellenlängen und zwei Polarisationskameras.

Fig. 15     zeigt schematisch in Form eines Blockdiagramms die polarisationsoptische Wirkungsweise einer beispielhaften Variante einer erfindungsgemäßen Visualisierungsanordnung unter Verwendung von zwei verschiedenen Wellenlängen.

Fig. 16     zeigt schematisch spektrale Auflösungen eines Prismas und eines Filters.

Fig. 17     zeigt schematisch ein erfindungsgemäßes Verfahren in Form eines Flussdiagramms.

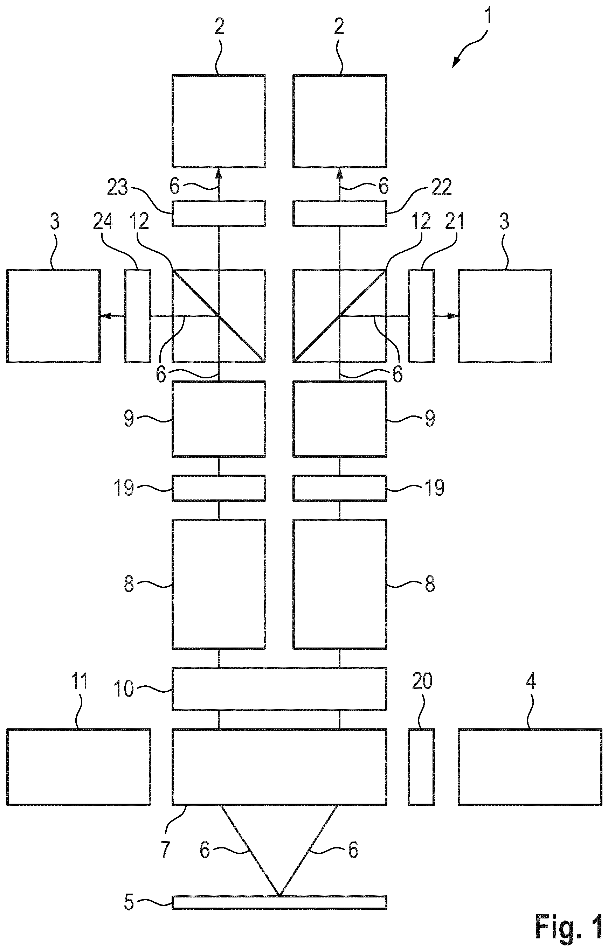

Die Figur 1 zeigt schematisch ein erfindungsgemäßes Operationsmikroskop 1 in Form eines Blockdiagramms. Das Operationsmikroskop 1 kann zum Beispiel zur Anwendung im Rahmen der Neuro- und Wirbelsäulenchirurgie ausgelegt sein. Das Operationsmikroskop 1 umfasst eine Visualisierungsanordnung mit einer Beleuchtungseinrichtung 4, einer Objektebene oder einem Objektraumbereich 5, in welchem beispielsweise ein vergrößert abzubildendes Objekt angeordnet sein kann, einer stereoskopischen Abbildungsvorrichtung und einer Polarisationsbestimmungsanordnung. Die Beleuchtungseinrichtung 4 ist zur Beleuchtung des Objektraumbereichs 5, also zur Einstrahlung von Lichtwellen auf ein Objekt, ausgelegt. Der Strahlengang ist durch Pfeile mit der Bezugsziffer 6 gekennzeichnet.

Das Mikroskop 1 ist vorzugsweise als vollständig digitales Mikroskop ausgebildet. Es ist bevorzugt dazu ausgelegt, sowohl Weißlicht-Videodaten als auch Fluoreszenz-Videodaten stereoskopisch in Echtzeit aufzunehmen und anzuzeigen. Dies wird die Verwendung von jeweils zwei Videokameras 2, welche zum Erfassen von Lichtwellen einer Mehrzahl an Lichtwellenlängen im sichtbaren Wellenlängenbereich ausgelegt sind (VIS-Kameras, z.B. RGB-Kameras), und mindestens einer weiteren Videokamera 3, bevorzugt wie gezeigt zwei weiteren Videokameras 3, welche zur Erhöhung der Empfindlichkeit als monochrome Kameras ausgestaltet und zum Erfassen von Fluoreszenzlicht ausgelegt ist/sind (Fluo-Kameras), erreicht. Die beiden VIS-Kameras 2 sind bevorzugt entweder 3-Chip oder 1-Chip Kameras. Die Fluo-Kameras 3 können auch zur Erfassung von Lichtwellen über den sichtbaren Bereich hinaus ausgelegt sein.

Zusätzlich enthält das Mikroskop 1 eine Optik mit variabler Brennweite (Varioskop) 7, zwei Zoom-Optiken 8, Iris-Elemente 19 sowie zwei Video-Objektive 9. Dabei sind die Optik mit variabler Brennweite (Varioskop) 7, zwei Zoom-Optiken 8, sowie zwei Video-Objektive 9 im Strahlengang 6 zwischen dem Objektraumbereich 5 oder einem im Objektraumbereich angeordneten Objekt und den genannten Videokameras 2, 3 angeordnet. Eine Laserautofokusvorrichtung 10 ist in der Figur 1 im Strahlengang 6 zwischen der Optik mit variabler Brennweite 7 und den Zoom-Optiken 8 angeordnet. Zwischen den Video-Objektiven 9 und den genannten Videokameras 2 und 3 sind im Strahlengang 6 jeweils Strahlteiler 12 angeordnet. Durch die Strahlteiler 12 werden individuelle Teilstrahlengänge erzeugt, sodass jeder der genannten Videokameras 2 und 3 im Strahlengang 6 ein individueller Teilstrahlengang zugeordnet ist. Das Mikroskop 1 enthält zudem optional eine Umfeldkamera 11, mit der ein großes Feld um den mittels des Mikroskops erfassten Objektbereich, z.B. den vergrößerten Operations-Situs, herum erfasst werden kann und die für Tooltracking und Navigationsfunktionen eingesetzt werden kann.

Die Polarisationsbestimmungsanordnung umfasst neben den oben genannten vier Videokameras 2, 3 eine Mehrzahl an Polarisationsfiltereinrichtungen 20-24 und eine nicht gezeigte Auswertungseinrichtung. Dabei ist jeweils eine Polarisationsfiltereinrichtung 21-24 im Strahlengang 6 vor jeder der vier genannten Videokameras 2, 3 angeordnet und eine Polarisationsfiltereinrichtung 20 im Strahlengang 6 nach der Beleuchtungseinrichtung 4 und vor dem Objektraumbereich 5 angeordnet. Dabei sind die Polarisationsfiltereinrichtungen 21-24 so eingestellt oder einstellbar, dass sich die im Strahlengang 6 vor mindestens einer, vorzugsweise beider, der zwei genannten weiteren Videokameras 3 und mindestens einer, insbesondere beider, der genannten, zum Erfassen von Lichtwellen einer Mehrzahl an Lichtwellenlängen im sichtbaren Wellenlängenbereich ausgelegten Videokameras 2 angeordneten Polarisationsfilter 21-24 in ihrer Polarisationswirkung jeweils voneinander unterscheiden. Mit anderen Worten unterscheidet sich im Betrieb die Einstellung der Polarisation mindestens einer Polarisationseinrichtungen 22, 23, welche vor einer der zum Erfassen von sichtbarem Licht ausgelegten Videokameras (VIS-Kamera) 2 angeordnet ist, von der Einstellung der Polarisation mindestens einer Polarisationseinrichtung 21, 24, welche vor einer der weiteren Videokameras 3 (z.B. Fluo-Kamera) angeordnet ist.

Die Polarisationsbestimmungsanordnung ermöglicht eine Analyse des Polarisationszustandes des von einem Objekt 5 abgestrahlten Lichts, wobei mithilfe der Mehrzahl an Videokameras 2, 3 eine Mehrzahl an Polarisationsmessungen gleichzeitig durchgeführt werden können. Dies erlaubt eine Erfassung und Auswertung der erforderlichen Daten in Echtzeit. Zum Erzeugen eines polarisationsaufgelösten, z.B. dreidimensionalen stereoskopischen, Gesamtbildes eines Objekts können zum Beispiel verschieden eingestellte Polarisationsfilterräder oder verschiedene Polarisatoren 20-24 vor den vier Kameras 2, 3 und im Strahlengang 6 nach der Beleuchtungseinrichtung 4 positioniert sein. Diese sind zum einen für die oben erwähnten Fluoreszenzoptionen mit passenden Anregungs- und Beobachtungsfiltern bestückt, zum anderen enthalten sie für die Polarimetrie geeignete Polarisatoren.

Im Falle der Verwendung einer eingangs beschriebenen alternativen erfindungsgemäßen Visualisierungsanordnung sind die Videokameras 2 als Polarisationskameras ausgestaltet. In dieser Variante kann auf die weiteren Videokameras 3, die Strahlteiler 12 und die Polarisationsfilteranordnungen 20 bis 24 verzichtet werden.

Im Folgenden werden für die polarisationsoptische Beschreibung des Systems vereinfachte Diagramme verwendet. Die Figur 2 zeigt schematisch in Form eines Blockdiagramms die polarisationsoptische Wirkungsweise einer erfindungsgemäßen Visualisierungsanordnung. Dabei erfolgt mittels einer Auswertungseinrichtung 13 eine Analyse und Auswertung der mittels der vier Kameras 2, 3 erfassten Bilddaten und eine Synthese dieser zu einem dreidimensionalen Gesamtbild mit Polarisationskontrast. Das erzeugte Gesamtbild wird mittels einer stereoskopischen Abbildungsvorrichtung 14 visualisiert bzw. einem Anwender angezeigt. Die Datenübertragung zwischen den Videokameras 2, 3 und der Auswertungseinrichtung 13 sowie zwischen der Auswertungseinrichtung 13 und der stereoskopischen Abbildungsvorrichtung 14 ist mit der Bezugsziffer 15 gekennzeichnet. Im Rahmen der Analyse und Auswertung der erfassten Daten werden bevorzugt Stokes-Vektoren oder Teile dieser ermittelt und eine Mehrzahl, vorzugsweise mehr als 4, Müller-Matrixkoeffizienten ermittelt, insbesondere berechnet. Mithilfe der ermittelten Müller-Matrixkoeffizienten wird der Polarisationskontrast ermittelt.

Die Figur 3 zeigt schematisch beispielhafte Polarisationsfilter und deren Bezeichnung. Dabei wird die Polarisationswirkung der Filter durch die Richtung der transmittierten Polarisation angezeigt. Die Polarisatoren 20-24 umfassen jeweils eine Anzahl, vorzugsweise eine Mehrzahl, an unterschiedlichen Polarisatoren, zum Beispiel einen oder mehrere der in der Figur 3 gezeigten Polarisatoren. Bevorzugt ist die Polarisation an jeder der Polarisationsfiltereinrichtungen individuell einstellbar.

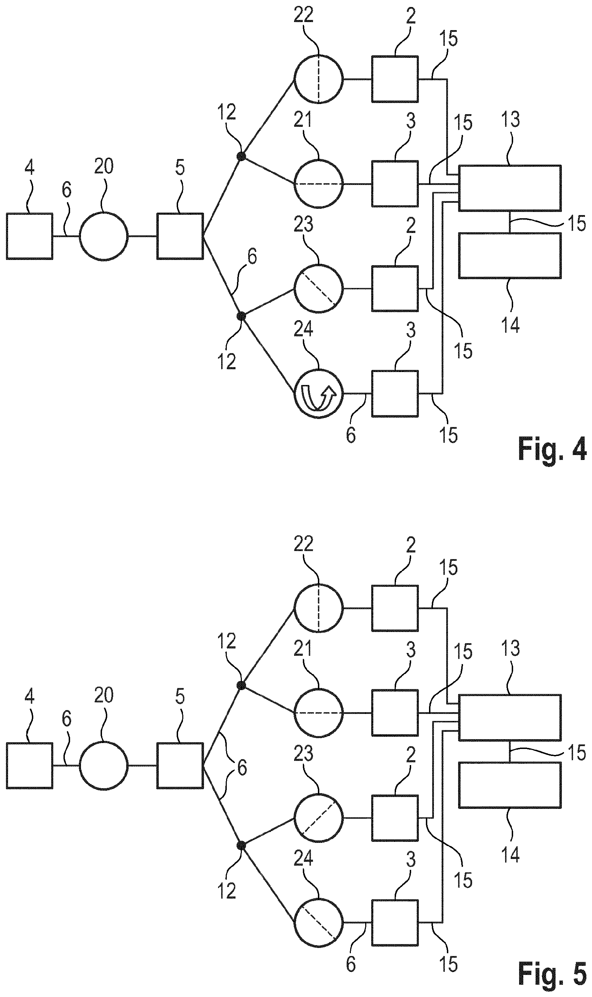

Für die Messung des vollständigen Stokes-Vektors benötigt man vier unterschiedliche Polarisatoren an den Filterpositionen 21-24. Ein Beispiel hierfür ist in der Figur 4 gezeigt, wobei die Polarisatoren auch anders auf die vier Filterpositionen 21-24 aufgeteilt sein können. In der Operationsmikroskopie ist möglicherweise hauptsächlich der lineare Polarisationsanteil von Interesse, sodass gegebenenfalls auf den zirkularen Polarisator verzichtet werden kann. Dieser könnte dann, wie in der Figur 5 gezeigt, zum Beispiel durch einen 135° Filter ersetzt werden, um Redundanz bei der Bestimmung der Gesamtintensität zu erhalten.

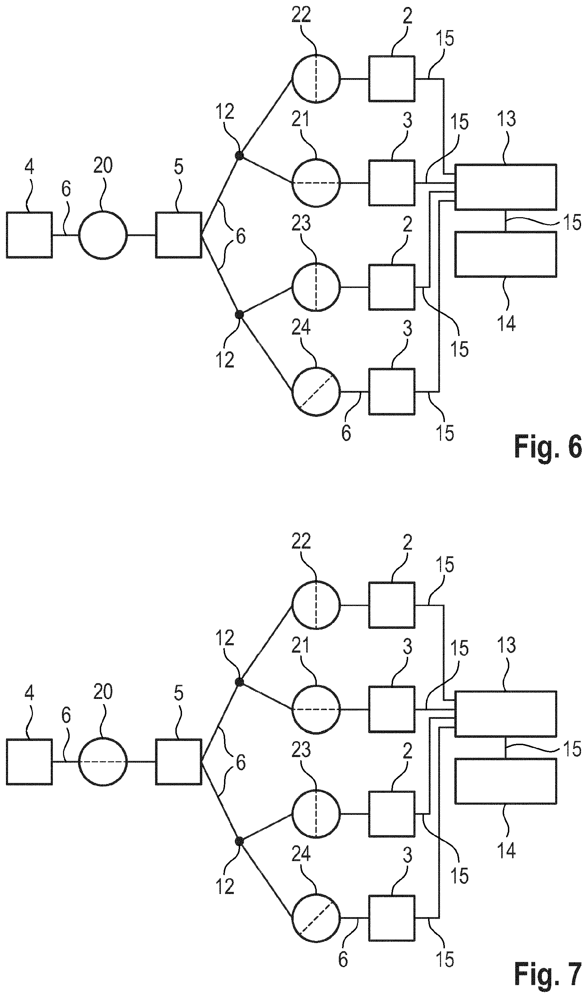

In einer weiteren, in der Figur 6 gezeigten Variante sind vor den beiden Videokameras für sichtbares Licht 2 (VIS-Kameras bzw. RGB Kameras) die gleichen Polarisationsfilter angeordnet oder eingestellt, um die Stereoabbildung nicht zu stören. Hierbei ist es vorteilhaft, wenn die Beleuchtungspolarisation 4, 20 orthogonal zur Polarisation des Filters 22, 23 vor den beiden Videokameras für sichtbares Licht 2 eingestellt ist. Dies ist in der Figur 7 gezeigt, wobei die Stokes-Vektorkomponenten S0, S1 und S2 mit ungestörter Stereoabbildung gemessen werden können.

Die Messung von Stokesparametern kann in einem digitalen Operationsmikroskop auch durch den Einsatz einer oder mehrerer Polarisationskameras erfolgen, die die notwendigen vier Messungen für die Ermittlung des Stokes-Vektors durch kleine Polarisationsfilter auf den Kamerapixeln realisiert. Dann ist wenigstens eine der vier Videokameras 2, 3 eine Polarisationskamera 17. Bevorzugt ist eine der weiteren Videokameras 3 eine Polarisationskameras, wie zum Beispiel in der Figur 8 dargestellt. Die Polarisationsfilteranordnung 24 in der Figur 8 ist dabei optional. Das Prinzip einer Polarisationskamera ist in der Figur 9 gezeigt. Vier in einer Ebene aneinander angrenzende Pixel 16 sind mit jeweils voneinander abweichenden Polarisatoren versehen.

Verfügbare Polarisationskameras, z.B. des Herstellers Sony, haben 5Mpixel und erreichen 23frams-per-second. Das bedeutet eine Zeitverzögerung von 43ms zwischen 2 Bildern, liegt also knapp in der angestrebten Spezifikation von 50ms. Die 4 Polarisatoren entsprechen in Gleichung (1) $P_0$, $P_{45}$, $P_{90}$, und $P_{135}$. Damit können die ersten 3 Stokesparameter ($S_0$, $S_1$, $S_2$) gemessen, aber nicht die 4. Komponente $S_3$. Für die Abbildung von Gewebe in Reflexion kann das eine erlaubte Einschränkung sein, weil die Strukturmerkmale im Wesentlichen linear sind, also auch primär die linearen Polarisationskomponenten beeinflussen sollten. Kritisch könnte werden, dass bei tiefer Gewebeabbildung Faserschichten schräg zueinander orientiert übereinander liegen. Das entspräche einer Kombination aus unterschiedlich orientierten linearen Verzögerungsplättchen, die zu einer Rotation und damit auch zu einer zirkulären Doppelbrechung führen können. Um alle Stokesparameter zu messen, kann z.B. der 135° Polarisator oder der 45° Polarisator in der Figur 5 noch mit einem Lambda/4-Retarder unter 0° überlagert werden. Beide zusammen bilden dann einen zirkularen Polarisator.

Bei einer unvollständigen Messung der Müllermatrix ist eine eingangs beschriebene Zerlegung nach Chipman und Lu nicht möglich. Außerdem ist es nicht das zwingende Ziel eines Operationsmikroskops mit Polarisationskontrast, die Müllermatrix vollständig und präzise zu messen, sondern dem Anwender, zum Beispiel dem Chirurgen, einen guten Gewebekontrast zu Verfügung zu stellen. Hierzu hat es in der Vergangenheit eine Vielzahl von Analysen gegeben.

Eine Variante ist in [6] beschrieben. Dort sind basierend auf Simulationen der Polarisationswirkung von isotropem und anisotropem Gewebe eine Reihe von Parametern definiert worden, die sich unmittelbar aus den Müllermatrix-Koeffizienten ergeben. In dem Fall ist es also ausreichend, einen endlichen Satz von Müllermatrix-Koeffizienten zu messen und daraus einen abgeleiteten Kontrast zu berechnen. Beispiele hierfür sind aus [6] in der folgenden Tabelle zusammengefasst:

| Kontrast | Berechnung | |
|---|---|---|
| Lineare Depolarisation | $$b = \frac{M_{11} + M_{22}}{M_{00}}$$ | (17) |
| Anisotrope Streuung | $$t_1 = \frac{\sqrt{(M_{11} - M_{22})^2 + (M_{12} + M_{21})^2}}{M_{00}}$$ | (18) |

(fortgesetzt)

| Kontrast | Berechnung | |
|---|---|---|
| Anisotrope Streuung | $$t_2 = \frac{\sqrt{M_{10}^2 + M_{20}^2}}{M_{00}}$$ | (19) |
| Doppelbrechung | $$t_3 = \frac{\sqrt{M_{31}^2 + M_{32}^2}}{M_{00}}$$ | (20) |

Das sind aber nur Beispiele. Es gibt noch weitere sinnvolle Kombinationen von Müllermatrix-Elementen, die sich als Bildkontrast der Operationsmikroskopie nutzen lassen.

Ein in [6] untersuchtes linear anisotropes Medium, welches in seiner Form gut mit biologischen Fasern vergleichbar, zeigt die folgende Symmetrie der Müllermatrix:

$$M = \begin{pmatrix} I_0 & A & B & 0 \\ A & C & D & 0 \\ B & D & E & 0 \\ 0 & 0 & 0 & 0 \end{pmatrix} \qquad (21)$$

Nur ein 3x3 Subset ist von Null verschieden und außerdem noch symmetrisch. Damit gibt es nur die 6 unabhängigen Komponenten $I_0, A, B, C, D, E$, welche zu bestimmen sind.

Um Teile der Müllermatrix zu messen, darf nicht nur ein Polarisationszustand eingestrahlt werden, sondern mehrere. Eine naheliegende Lösung wäre, den Filter 20 rotierbar zu gestalten oder durch einen ferroelektrischen Flüssigkristallfilter zu ergänzen. Allerdings wird damit die Beleuchtung mit mehreren Polarisationszuständen nur zeitsequenziell möglich. Da bereits die polarisierte Abbildung nur knapp in der angestrebten Spezifikation liegt, dürfte eine zeitsequenzielle polarisierte Beleuchtung die Anforderung von max. 50ms Zeitverzögerung für den Gewebekontrast deutlich überschreiten. Wenn die Zeit nicht als differenzierender Parameter für die Beleuchtungspolarisation eingesetzt werden kann, kann im Rahmen der vorliegenden Erfindung die Wellenlänge des Beleuchtungslichtes verwendet werden. Eine gleichzeitige Beleuchtung mit zwei Polarisationszuständen wird möglich, wenn diese an verschiedenen Stellen im Spektrum angeordnet sind. Dann lassen sich die zugehörigen Bilder mit einem Spektralfilter auch wieder rekonstruieren. Das heißt, die Beleuchtung muss in zwei schmalen spektralen Bändern unterschiedlich polarisiert sein. Wenn trotzdem ein "normaler Farbeindruck" entstehen soll, sollte darum herum unpolarisertes oder in einem beliebigen Polarisationszustand befindliches Licht vorliegen (siehe Figur 3). Ansonsten besteht die Option einer bichromatischen Beleuchtung. Das kann zum Beispiel durch 2 Notch-Filter realisiert werden, wie in Figur 10 gezeigt.

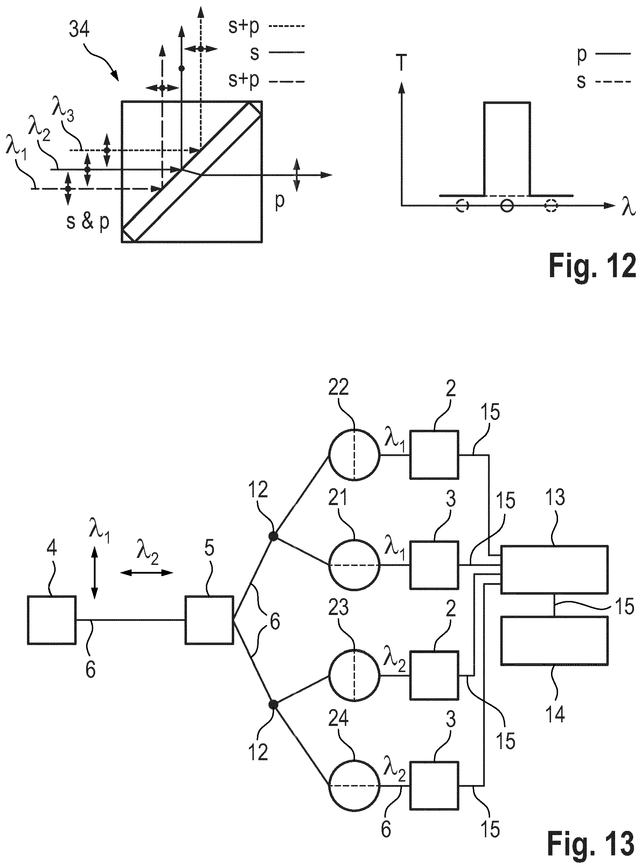

Die Figur 10 zeigt schematisch 2 Diagramme, welche mögliche Beleuchtungen des Objektraumbereichs darstellen. **In** beiden Diagrammen ist die Intensität I des eingestrahlten Lichts in Abhängigkeit von der Wellenlänge $\lambda$ dargestellt. Das obere Diagramm zeigt eine bichromatische Beleuchtung mit einem ersten Wellenlängenbereich 31 und einem zweiten Wellenlängenbereich 32. Die beiden Wellenlängenbereiche 31 und 32 sind abweichend voneinander polarisiert. Das untere Diagramm zeigt eine polychromatische Beleuchtung, wobei die beiden Wellenlängenbereiche 31 und 32 abweichend voneinander polarisiert sind und der übrige Spektralbereich 33 unpolarisiert ist. Dies kann zum Beispiel durch zwei Notch-Filter realisiert werden, wie in der Figur 11 gezeigt. Dabei werden durch Transmission von unpolarisiertem Licht durch zwei schmalbandige polarisierende Notchfilter zwei schmalbandig polarisierte Spektralteile 31 und 32 realisiert. Zur Realisierung des in der Figur 11 gezeigten Konzeptes können z.B. polarisierende Bandpassfilter in Reflexion eingesetzt werden, wie in der Figur 12 gezeigt. Die Figur 12 veranschaulicht die Wirkungsweise eines polarisierenden Bandpassfilters 34 von Semrock (https://www.semrock.com/a-new-class-of-polarization-optics-designed-specifically-for-lasers.aspx). Links ist der Strahlengang für drei verschiedene Wellenlängen $\lambda_1$, $\lambda_2$ und $\lambda_3$ sowie die jeweilige linearen Polarisationen s und p gezeigt. Dabei kennzeichnen s und p zueinander orthogonale lineare Polarisationen. Rechts in der Figur 12 ist ein Diagramm gezeigt, welches die Transmission T durch den Filter 34 in Abhängigkeit von der Wellenlänge $\lambda$ und der Polarisation s und p abbildet.

Je nachdem, welche Polarisationszustände eingestrahlt werden und welche gemessen werden, sind verschiedene Müllermatrix-Komponenten der

Messung zugänglich, mit der in Figur 13 gezeigten Konfiguration z.B. $M_{00}, M_{01}, M_{10}, M_{11}$. In der in der Figur 13 gezeigten Variante wird mit zwei zueinander senkrecht polarisierten Wellenlängen oder Wellenlängenbereichen $\lambda_1$ und $\lambda_2$ eingestrahlt, wobei die einzelnen polarisierten Wellenlängen von unterschiedlichen Kameras analysiert werden, in der gezeigten Variante die Wellenlänge $\lambda_1$ von einer ersten Videokamera für sichtbares Licht (RGB-Kamera) 2 und einer ersten weiteren Videokamera 3 und die Wellenlänge $\lambda_2$ von einer zweiten Videokamera für sichtbares Licht (RGB-Kamera) 2 und einer zweiten weiteren Videokamera 3.

Selbstverständlich sind andere Konfigurationen denkbar, z.B. können die RGB Kameras 2 durch monochrome Kameras 3 ersetzt werden oder umgekehrt, es können aber ohne Polarisationskameras wegen der spektralen Aufteilung nicht 3 oder 4 sondern nur 2 Stokes-Vektor-Komponenten gemessen werden, in der in der Figur 13 gezeigten Variante S0 und S1. Das könnten aber auch S0 und S2 sein, was die Müllermatrix-Komponenten $M_{00}, M_{02}, M_{20}, M_{22}$ zugänglich macht.

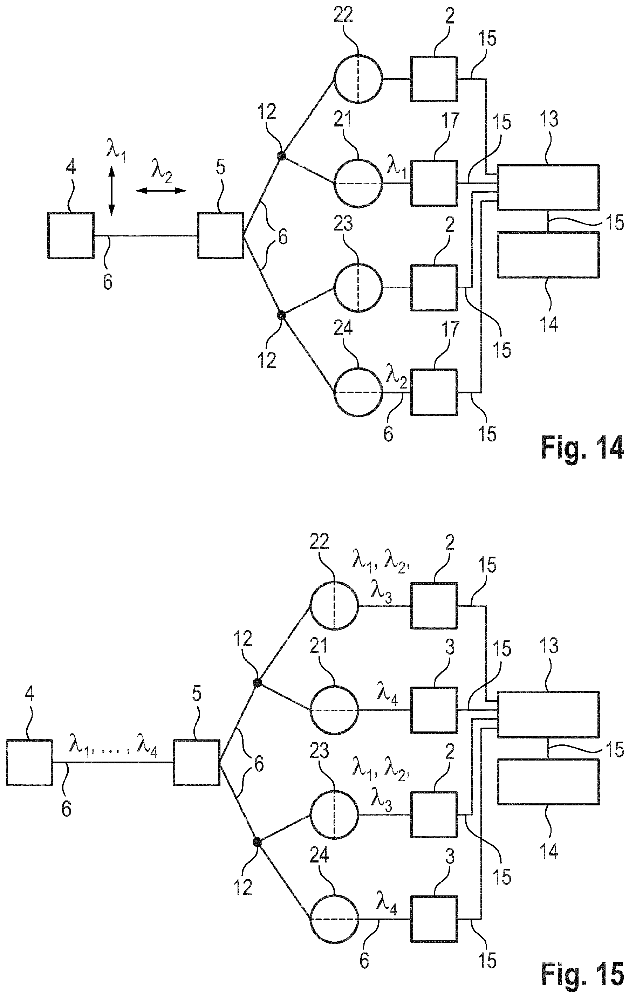

In der in der Figur 14 gezeigten Variante kann mit 2 Polarisationskameras 17 als weitere Kameras 3 und zwei Eingangspolarisationen die halbe Müllermatrix, also 8 Müllermatrixelemente, gemessen werden. Hierbei ist der nicht gezeichnete Filter 20 ein polarisierender Notchfilter, der in einem unpolarisierten Beleuchtungsspektrum lediglich die beiden Wellenlängen $\lambda_1$ und $\lambda_2$ polarisiert. Der Rest des Beleuchtungsspektrums ist unpolarisiert. Filter 21 ist ein Filter für die Wellenlänge $\lambda_1$ und optional der Polarisation 1 und Filter 24 ist ein Bandpassfilter für die Wellenlänge $\lambda_2$ und optional der Polarisation 2. Alternativ könnte auch der neutrale Strahlteiler als dichroitischer Strahlteiler ausgebildet sein, z.B. ein erster für eine Wellenlänge mit einer ersten Polarisation und ein zweiter für Wellenlänge mit einer zweiten Polarisation. Das wäre für das Licht-Budget effizienter, kostet aber Flexibilität. Der Vorteil dieses Verfahrens ist, dass die Polarisation eine Zusatzoption wird, welche die Standardabbildung nicht stört.

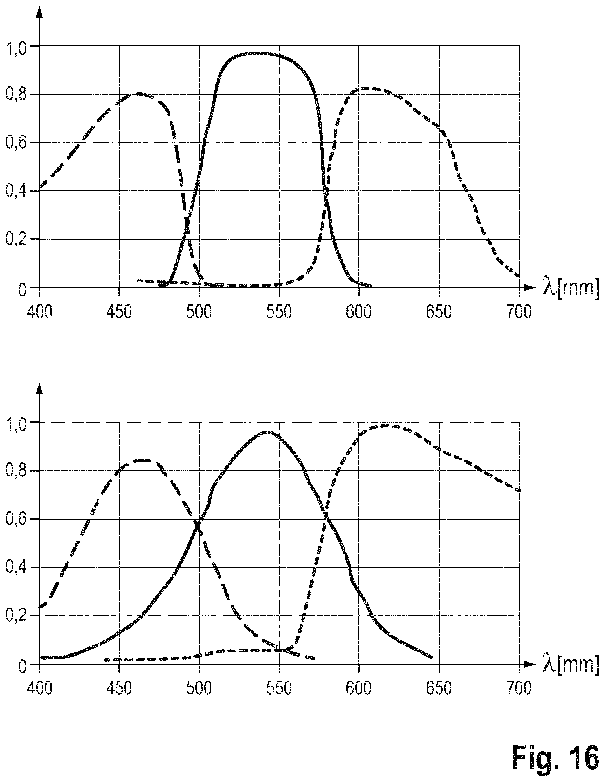

Figur 15 zeigt eine weitere Ausführungsform für eine spektrale Kodierung, bei der die spektralen Eigenschaften der RGB-Kanäle der beiden Farbvideokameras 2 vorteilhaft verwendet werden. Wie zu Figur 14 beschrieben ist auch in der Figur 15 der nicht gezeichnete Filter 1 ein polarisierender Notch-Filter, der jetzt so ausgelegt ist, dass mit 4 unterschiedlichen Wellenlängen mit definierten, vorgegebenen Polarisationen beleuchtet wird. Die Polarisationen für die verschiedenen Wellenlängen können unterschiedlich oder teilweise identisch sein. Als Videokameras 2 (RGB-Kameras) können entweder 1-Chip oder 3-Chip-Kameras eingesetzt werden. Im Falle von 1-Chip-Kameras trennt ein Bayer-Filter den einen Sensor in 3 spektral getrennte Pixelarrays, während bei 3-Chip-Kameras das verwendete Teilerprisma die spektrale Aufspaltung auf die drei Sensoren ermöglicht. Die Auswahl der Wellenlängen des Filters 20 erfolgt in Figur 15 so, dass die RGB-Sensoren die Wellenlängen getrennt detektieren und es kein Übersprechen der verschiedenen Wellenlängen gibt. Dies kann z.B. durch Beleuchtung mit den Wellenlängen 400nm, 540nm, 680nm erreicht werden, wie die typischen spektralen Sensitivitäten der RGB-Sensoren und Bayer-Filter in Figur 16 zeigen. In der Figur 16 ist die relative spektrale Sensitivität in Abhängigkeit von der Wellenlänge eines Teilerprismas (oben) und eines Bayer-Filters (unten) gezeigt. Die 4. Wellenlänge des Beleuchtungsfilters 20 ist so gewählt, dass die beiden Monochrome-Kameras 3 spektral getrennt von den RGB-Kameras 2 Signale detektieren können. Dies kann z.B. mit Beleuchtung einer Wellenlänge im infraroten Wellenlängenbereich erreicht werden, bei der die RGB-Kameras 2 nicht mehr sensitiv sind (z.B. bei 800nm). Natürlich ist es auch denkbar, dass die Wellenlängen anders gewählt sind und störende Wellenlängen vor den Sensoren durch geeignete Notch-Filter entfernt werden. Die Filter 22 und 23 vor den RGB-Kameras 2 sind somit idealerweise als Multibandpass-Filter für die Wellenlängen 400nm, 540nm und 680nm und ausgelegt. Gleichzeitig können die Filter 22 und 23 unterschiedlich bezüglich ihrer Polarisationseigenschaften ausgelegt sein. Die Filter 21 und 24 vor den weiteren Videokameras (Monochrome-Kameras) 3 sind als BandpassFilter für die Wellenlänge 800nm ausgelegt, mit ebenfalls optional unterschiedlicher Polarisationseigenschaft bezüglich der Filter 21 und 24.

Das Ausführungsbeispiel der Figur 15 beschreibt einen Spezialfall und kann wie folgt verallgemeinert werden: Im ersten Schritt wird die Anzahl N der im System vorhandenen Detektionskanäle bestimmt. Im in der Figur 1 gezeigten Beispiel sind dies 8 Kanäle, wobei die beiden weiteren Videokameras 3 (Monochrome-Kameras) zwei und die beiden Videokameras für sichtbares Licht 2 (RGB-Kameras) zusätzlich sechs Kanäle liefern. Das System wird nun so ausgelegt, dass diese N Detektionskanäle einen für die jeweilige Anwendung optimalen Gewebekontrast liefern. Dies wird dadurch erreicht, dass sich alle N Kanäle zumindest in einer Eigenschaft, z.B. in Spektrum und/oder Polarisation - unterscheiden.

Ein erstes mögliches Ausführungsbeispiel für ein System mit 8 Detektionskanälen ist eine Beleuchtung mit 8 unterschiedlichen Polarisationen, die spektral getrennt sind, und eine Detektion mit 8 Detektoren, die mit einer Polarisation spektral getrennt analysieren. Ein zweites mögliches Ausführungsbeispiel für ein System mit 8 Detektionskanälen ist eine Beleuchtung mit 2 definierten Polarisationen, die spektral getrennt sind und eine Detektion mit 8 Detektoren, wobei jeweils 4 Detektoren einen Spektralbereich mit 4 unterschiedlichen Analysatoren analysieren.

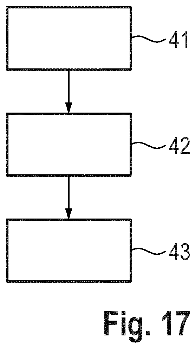

Die Figur 17 zeigt schematisch ein erfindungsgemäßes Verfahren zum Erzeugen einer Abbildung, bevorzugt einer stereoskopischen Abbildung, mit Polarisationskontrast eines Objekts mittels einer zuvor beschriebenen erfindungsgemäßen Visualisierungsanordnung, beispielsweise einer Visualisierungsanordnung eines Operationsmikroskops, in Form eines Flussdiagramms. Das Verfahren umfasst in Schritt 41 das Einstrahlen von Lichtwellen auf das Objekt mittels der Beleuchtungseinrichtung, in Schritt 42 das Erfassen von Lichtwellen, welche nach einer Wechselwirkung des Objekts mit den eingestrahlten Lichtwellen von dem Objekt abgestrahlt werden, mittels der mindestens drei Videokameras oder der mindestens zwei Polarisationskameras, und in Schritt 43 das Erzeugen einer vorzugsweise stereoskopischen Abbildung mit Polarisationskontrast basierend auf einem generierten Polarisationszustand der eingestrahlten Lichtwellen und/oder einem analysierten Polarisationszustand der erfassten Lichtwellen. Zu konkreten Ausführungsvarianten des Verfahrens wird auf die Ausführungen zu den Figuren 1 bis 16 verwiesen.

Bezugszeichenliste:

| | |
|---|---|
| 1 | Operationsmikroskop mit Visualisierungsanordnung |
| 2 | Videokamera |
| 3 | Videokamera |
| 4 | Beleuchtungseinrichtung |
| 5 | Objektbereich, Objekt |
| 6 | Strahlengang |
| 7 | Optik mit variabler Brennweite (Varioskop) |
| 8 | Zoom-Optik |
| 9 | Video-Objektiv |
| 10 | Laserautofokusvorrichtung |
| 11 | Umfeldkamera |
| 12 | Strahlteiler |
| 13 | Auswertungseinrichtung |
| 14 | (stereoskopische) Abbildungsvorrichtung |
| 15 | (Bild)Datenübertragung |
| 16 | Pixel |
| 17 | Polarisationskamera |
| 19 | Iris-Element |
| 20 | Polarisationsfiltereinrichtung |
| 21 | Polarisationsfiltereinrichtung |
| 22 | Polarisationsfiltereinrichtung |
| 23 | Polarisationsfiltereinrichtung |
| 24 | Polarisationsfiltereinrichtung |
| 31 | Wellenlängenbereich |
| 32 | Wellenlängenbereich |
| 33 | Wellenlängenbereich |
| 34 | polarisierender Bandpassfilter |
| 41 | Einstrahlen von Lichtwellen |
| 42 | Erfassen von Lichtwellen, welche nach einer Wechselwirkung des Objekts mit den eingestrahlten Lichtwellen von dem Objekt abgestrahlt werden |
| 43 | Erzeugen einer Abbildung mit Polarisationskontrast basierend auf einem generierten Polarisationszustand der eingestrahlten Lichtwellen und/oder einem analysierten Polarisationszustand der erfassten Lichtwellen |
| I | Intensität |
| T | Transmission |
| λ | Wellenlänge |
| s | lineare Polarisation |
| p | lineare Polarisation |
| P0 | Polarisator unter 0 Grad |
| P90 | Polarisator unter 90 Grad |
| P45 | Polarisator unter 45 Grad |
| P135 | Polarisator unter 135 Grad |
| PC | zirkularer Polarisator |
| PN | neutraler Polarisator |

**Patentansprüche**

1. Visualisierungsanordnung (1) für die Mikrochirurgie, welche eine Abbildungsvorrichtung (14), eine Beleuchtungseinrichtung (4) und eine Polarisationsbestimmungsanordnung umfasst,
**dadurch gekennzeichnet, dass**

   die Polarisationsbestimmungsanordnung mindestens zwei Videokameras (2) zum Erfassen von mehrfarbigem sichtbarem Licht und mindestens eine weitere Videokamera (3), wobei jeder der drei genannten Videokameras (2, 3) im Strahlengang (6) ein individueller Teilstrahlengang zugeordnet ist, eine Mehrzahl an Polarisationsfiltereinrichtungen (20-24) und eine Auswertungseinrichtung (13) umfasst,

wobei mindestens jeweils eine Polarisationsfiltereinrichtung (21-24) im Strahlengang (6) vor drei der mindestens drei Videokameras (2, 3) angeordnet ist,

wobei die Polarisationsfiltereinrichtungen (20-24) so eingestellt oder einstellbar sind, dass sich die im Strahlengang (6) vor der mindestens einen genannten weiteren Videokamera (3) und mindestens einer der genannten Videokameras (2) zum Erfassen von mehrfarbigem sichtbarem Licht angeordneten Polarisationsfilter (20-24) in ihrer Polarisationswirkung voneinander unterscheiden,

wobei die Auswertungseinrichtung (13) dazu ausgelegt ist, mittels der von den Videokameras (2, 3) erfassten Bilder Abbildungen mit Polarisationskontrast zu erzeugen und mittels der Abbildungsvorrichtung (14) anzuzeigen.

2.  Visualisierungsanordnung (1) nach Anspruch 1,
    **dadurch gekennzeichnet, dass**
    die Auswertungseinrichtung (13) dazu ausgelegt ist, die erzeugten Abbildungen mit Polarisationskontrast mittels der Abbildungsvorrichtung (14) mittels mindestens zwei Videokameras (2) zum Erfassen von mehrfarbigem sichtbarem Licht erfassten Weißlicht-Abbildungen überlagert anzuzeigen.

3.  Visualisierungsanordnung (1) nach Anspruch 1 oder 2,
    **dadurch gekennzeichnet, dass**

    mindestens eine Polarisationsfiltereinrichtung (20) im Strahlengang (6) nach der Beleuchtungseinrichtung (4) und vor einem Objektraumbereich (5) angeordnet ist
    und/oder
    mindestens eine der Polarisationsfiltereinrichtungen (20-24) eine Mehrzahl sich voneinander unterscheidende Polarisatoren umfasst.

4.  Visualisierungsanordnung (1) nach einem der Ansprüche 1 bis 3,
    **dadurch gekennzeichnet, dass**
    die mindestens eine weitere Videokamera (3) zum Erfassen von monochromen Licht ausgelegt ist oder mindestens eine der genannten Videokameras (2, 3) als Polarisationskamera ausgebildet ist.

5.  Visualisierungsanordnung (1) nach Anspruch 4,
    **dadurch gekennzeichnet, dass**
    mindestens eine vor einer Videokamera (2) zum Erfassen von mehrfarbigem sichtbarem Licht angeordnete Polarisationsfiltereinrichtung (22, 23) einen Zirkularpolarisator umfasst.

6.  Visualisierungsanordnung (1) einem der Ansprüche 1 bis 5,
    **dadurch gekennzeichnet, dass**
    die Auswertungseinrichtung (13) dazu ausgelegt ist, mittels der von den Videokameras (2, 3) erfassten Bilder einen Satz von Müllermatrix-Koeffizienten zu bestimmen und einen daraus abgeleiteten Polarisationskontrast zu bestimmen.

7.  Visualisierungsanordnung (1) einem der Ansprüche 1 bis 6,
    **dadurch gekennzeichnet, dass**

    eine im Strahlengang (6) nach der Beleuchtungseinrichtung (4) und vor einem Objektraumbereich (5) angeordnete Polarisationsfiltereinrichtung (20) dazu ausgestaltet ist, mindestens zwei festgelegte, voneinander abweichende Wellenlängen gleichzeitig voneinander abweichend zu polarisieren
    und/oder
    mindestens eine der Polarisationsfiltereinrichtungen (20-24) einen Wellenlängenfilter und/oder einen wellenlängenselektiven Polarisator umfasst.

8.  Visualisierungsanordnung (1) einem der Ansprüche 1 bis 7,
    **dadurch gekennzeichnet, dass**

    die Beleuchtungseinrichtung (4) zur Abstrahlung von bichromatischem Licht ausgelegt ist
    und/oder
    die Beleuchtungseinrichtung (4) zur Abstrahlung von polychromatischem Licht ausgelegt ist und eine im Strahlengang (6) nach der Beleuchtungseinrichtung (4) und vor einem Objektraumbereich (5) angeordnete Polarisationsfiltereinrichtung (20) mindestens einen Notch-Filter und/oder eine Anzahl an Bandpassfiltern umfasst

und/oder

**gekennzeichnet durch** mindestens einen Strahlteiler (12), der im Strahlengang (6) zwischen einem Objektraumbereich (5) und den Videokameras (2, 3) angeordnet ist.

9. Visualisierungsanordnung (1) für die Mikrochirurgie, welche eine Abbildungsvorrichtung (14), eine Beleuchtungseinrichtung (4) und eine Polarisationsbestimmungsanordnung umfasst,
**dadurch gekennzeichnet, dass**

die Polarisationsbestimmungsanordnung zwei Polarisationskameras (17), wobei jeder der zwei Polarisationskameras (17) im Strahlengang (6) ein individueller Teilstrahlengang zugeordnet ist, und eine Auswertungseinrichtung (13) umfasst,
wobei die Auswertungseinrichtung (13) dazu ausgelegt ist, mittels der von den Polarisationskameras (17) erfassten Bilder Abbildungen mit Polarisationskontrast zu erzeugen und mittels der Abbildungsvorrichtung (14) anzuzeigen, wobei
die Auswertungseinrichtung (13) dazu ausgelegt ist, die erzeugten Abbildungen mit Polarisationskontrast mittels der Abbildungsvorrichtung (14) Weißlicht-Abbildungen, welche mittels der zwei Polarisationskameras (17) erfasst werden, überlagert anzuzeigen.

10. Verfahren zum Erzeugen einer Abbildung mit Polarisationskontrast eines Objekts (5) mittels einer Visualisierungsanordnung (1) für die Mikrochirurgie gemäß einem der Ansprüche 1 bis 9,
**dadurch gekennzeichnet, dass**
das Verfahren folgende Schritte umfasst:

- Einstrahlen von Lichtwellen (41) auf das Objekt (5) mittels der Beleuchtungseinrichtung (4),
- Erfassen von Lichtwellen (42), welche nach einer Wechselwirkung des Objekts (5) mit den eingestrahlten Lichtwellen von dem Objekt (5) abgestrahlt werden, mittels der mindestens drei Videokameras (2, 3) oder der mindestens zwei Polarisationskameras (17),
- Erzeugen einer Abbildung mit Polarisationskontrast (43) basierend auf einem generierten Polarisationszustand der eingestrahlten Lichtwellen und/oder einem analysierten Polarisationszustand der erfassten Lichtwellen.

11. Verfahren nach Anspruch 10,
**dadurch gekennzeichnet, dass**

Lichtwellen eines definierten Polarisationszustandes mittels der Beleuchtungseinrichtung (4) und einer im Strahlengang (6) nach der Beleuchtungseinrichtung (4) und vor dem Objekt (5) angeordneten Polarisationsfiltereinrichtung (20) eingestrahlt werden
und/oder
der Polarisationszustand der empfangenen Lichtwellen mittels der mindestens drei Videokameras (2, 3) und der im Strahlengang (6) vor diesen angeordneten Polarisationsfiltereinrichtungen (21-24) und/oder mittels der Polarisationskameras (17) analysiert wird.

12. Verfahren nach einem der Ansprüche 10 bis 11,
**dadurch gekennzeichnet, dass**
mittels der Auswertungseinrichtung (13) eine Abbildung mit Polarisationskontrast basierend auf einem analysierten Polarisationszustand der erfassten Lichtwellen erzeugt wird, wobei mittels der von den Videokameras (2, 3) und/oder den Polarisationskameras (17) erfassten Bilder ein Satz von Müllermatrix-Koeffizienten bestimmt wird und ein daraus abgeleiteter Polarisationskontrast bestimmt wird.

13. Verfahren nach einem der Ansprüche 10 bis 12,
**dadurch gekennzeichnet, dass**
die Abbildung mit Polarisationskontrast innerhalb eines Zeitintervalls von weniger als 50ms erzeugt wird und/oder die Auflösung der erzeugten stereoskopischen Abbildung mit Polarisationskontrast weniger als 10 Prozent von der Auflösung einer entsprechenden Abbildung ohne Polarisationskontrast abweicht.

14. Verfahren nach Anspruch 13,
**dadurch gekennzeichnet, dass**

die Abbildung mit Polarisationskontrast innerhalb eines Zeitintervalls von weniger als 50ms erzeugt und einer mehrfarbigen Abbildung überlagert angezeigt wird.

15. Mikroskop, welches eine Visualisierungsanordnung (1) nach einem der Ansprüche 1 bis 9 umfasst oder dazu ausgelegt ist, ein Verfahren nach einem der Ansprüche 10 bis 14 auszuführen.

**Claims**

1. Visualization arrangement (1) for microsurgery, comprising an imaging apparatus (14), an illumination device (4) and a polarization-determining arrangement,
**characterized in that**

   the polarization-determining arrangement comprises at least two video cameras (2) for capturing multicoloured visible light and at least one further video camera (3), with each of the three aforementioned video cameras (2, 3) being assigned an individual partial beam path in the beam path (6), a plurality of polarization-filtering devices (20-24) and an evaluation device (13),
   wherein at least one polarization-filtering device (21-24) is arranged in the beam path (6) upstream of each of three of the at least three video cameras (2, 3),
   wherein the polarization-filtering devices (20-24) are set or settable such that the polarization filters (20-24) arranged in the beam path (6) upstream of the at least one specified further video camera (3) and at least one of the aforementioned video cameras (2) for capturing multicoloured visible light differ from one another in terms of their polarization effect,
   wherein the evaluation device (13) is designed to create image representations with polarization contrast using the images captured by the video cameras (2, 3) and to display said image representations by means of the imaging apparatus (14).

2. Visualization arrangement (1) according to Claim 1, **characterized in that**
   the evaluation device (13) is designed to display the created image representations with polarization contrast by means of the imaging apparatus (14) in a manner overlaid with white-light image representations captured by means of at least two video cameras (2) for capturing multicoloured visible light.

3. Visualization arrangement (1) according to Claim 1 or 2,
   **characterized in that**

   at least one polarization-filtering device (20) is arranged in the beam path (6) downstream of the illumination device (4) and upstream of an object space region (5)
   and/or
   at least one of the polarization-filtering devices (20-24) comprises a plurality of polarizers that differ from one another.

4. Visualization arrangement (1) according to any of Claims 1 to 3,
   **characterized in that**
   the at least one further video camera (3) is designed to capture monochrome light, or at least one of the aforementioned video cameras (2, 3) takes the form of a polarization camera.

5. Visualization arrangement (1) according to Claim 4, **characterized in that**
   at least one polarization-filtering device (22, 23) arranged upstream of a video camera (2) for capturing multicoloured visible light comprises a circular polarizer.

6. Visualization arrangement (1) according to any of Claims 1 to 5,
   **characterized in that**
   the evaluation device (13) is designed to use the images captured by the video cameras (2, 3) to determine a set of Mueller matrix coefficients and to determine a polarization contrast derived from said coefficients.

7. Visualization arrangement (1) according to any of Claims 1 to 6,
   **characterized in that**

a polarization-filtering device (20) arranged in the beam path (6) downstream of the illumination device (4) and upstream of an object space region (5) is configured to simultaneously polarize at least two defined, mutually deviating wavelengths in a manner deviating from one another
and/or
at least one of the polarization-filtering devices (20-24) comprises a wavelength filter and/or a wavelengthselective polarizer.

8. Visualization arrangement (1) according to any of Claims 1 to 7,
**characterized in that**

the illumination device (4) is designed to emit bichromatic light
and/or
the illumination device (4) is designed to emit polychromatic light, and a polarization-filtering device (20) arranged in the beam path (6) downstream of the illumination device (4) and upstream of an object space region (5) comprises at least one notch filter and/or a number of bandpass filters
and/or
**characterized by** at least one beam splitter (12) that is arranged in the beam path (6) between an object space region (5) and the video cameras (2, 3).

9. Visualization arrangement (1) for microsurgery, comprising an imaging apparatus (14), an illumination device (4) and a polarization-determining arrangement, **characterized in that**

the polarization-determining arrangement comprises two polarization cameras (17), with each of the two polarization cameras (17) being assigned an individual partial beam path in the beam path (6), and an evaluation device (13),
wherein the evaluation device (13) is designed to create image representations with polarization contrast using the images captured by the polarization cameras (17) and to display said image representations by means of the imaging apparatus (14), wherein
the evaluation device (13) is designed to display the created image representations with polarization contrast by means of the imaging apparatus (14) in a manner overlaid with white-light image representations captured by means of the two polarization cameras (17).

10. Method for creating an image representation with polarization contrast of an object (5) by means of a visualization arrangement (1) for microsurgery according to any of Claims 1 to 9,
**characterized in that**
the method comprises the following steps:

- radiating light waves (41) onto the object (5) by means of the illumination device (4),
- capturing light waves (42), which are emitted by the object (5) following an interaction of the object (5) with the radiated-in light waves, by means of the at least three video cameras (2, 3) or the at least two polarization cameras (17),
- creating an image representation with polarization contrast (43) on the basis of a generated polarization state of the radiated-in light waves and/or an analysed polarization state of the captured light waves.

11. Method according to Claim 10,
**characterized in that**

light waves in a defined polarization state are radiated-in by means of the illumination device (4) and a polarization-filtering device (20) arranged in the beam path (6) downstream of the illumination device (4) and upstream of the object (5)
and/or
the polarization state of the received light waves is analysed by means of the at least three video cameras (2, 3) and the polarization-filtering devices (21-24) arranged upstream of said video cameras in the beam path (6) and/or by means of the polarization cameras (17).

12. Method according to either of Claims 10 and 11,
**characterized in that**
the evaluation device (13) is used to create an image representation with polarization contrast on the basis of an analysed polarization state of the captured light waves, wherein a set of Mueller matrix coefficients is determined by means of the images captured by the video cameras (2, 3) and/or the polarization cameras (17), and a polarization contrast derived from said coefficients is determined.

**13.** Method according to any of Claims 10 to 12,
**characterized in that**
the image representation with polarization contrast is created within a time interval of less than 50 ms, and/or the resolution of the stereoscopic image representation with polarization contrast created deviates from the resolution of a corresponding image representation without polarization contrast by less than 10 percent.

**14.** Method according to Claim 13,
**characterized in that**
the image representation with polarization contrast is created within a time interval of less than 50 ms and displayed in a manner overlaid on a multicoloured image representation.

**15.** Microscope comprising a visualization arrangement (1) according to any of Claims 1 to 9 or designed to carry out a method according to any of Claims 10 to 14.

**Revendications**

**1.** Ensemble de visualisation (1) pour la microchirurgie, lequel comprend un dispositif d'imagerie (14), un dispositif d'éclairage (4) et un ensemble de détermination de polarisation,
**caractérisé en ce que**

l'ensemble de détermination de polarisation comprend au moins deux caméras vidéo (2) destinées à capturer une lumière visible multicolore, et au moins une autre caméra vidéo (3), chacune des trois caméras vidéo (2, 3) étant associée à un trajet de faisceau partiel individuel dans le trajet de faisceau (6), une pluralité de dispositifs à filtre de polarisation (20-24) et un dispositif d'évaluation (13),
dans lequel au moins un dispositif à filtre de polarisation (21-24) est respectivement disposé dans le trajet de faisceau (6) en amont de trois des au moins trois caméras vidéo (2, 3),
dans lequel les dispositifs à filtre de polarisation (20-24) sont réglés ou peuvent être réglés de telle sorte que les filtres de polarisation (20-24), qui sont agencés dans le trajet de faisceau (6) en amont de ladite au moins une caméra vidéo (3) et d'au moins l'une desdites caméras vidéo (2) destinées à capturer la lumière visible multicolore, diffèrent les uns des autres par leur effet de polarisation,
dans lequel le dispositif d'évaluation (13) est conçu pour générer des images à contraste de polarisation au moyen des images capturées par les caméras vidéo (2, 3) et pour les afficher au moyen du dispositif d'imagerie (14).

**2.** Ensemble de visualisation (1) selon la revendication 1,
**caractérisé en ce que**
le dispositif d'évaluation (13) est conçu pour afficher, au moyen du dispositif d'imagerie (14), les images générées à contraste de polarisation de manière superposée à des images en lumière blanche capturées au moyen d'au moins deux caméras vidéo (2) destinées à capturer une lumière visible multicolore.

**3.** Ensemble de visualisation (1) selon la revendication 1 ou 2,
**caractérisé en ce que**

au moins un dispositif à filtre de polarisation (20) est disposé dans le trajet de faisceau (6) en aval du dispositif d'éclairage (4) et en amont d'une zone d'espace objet (5)
et/ou **en ce que**
au moins l'un des dispositifs à filtre de polarisation (20-24) comprend une pluralité de polariseurs différents les uns des autres.

**4.** Ensemble de visualisation (1) selon l'une des revendications 1 à 3,
**caractérisé en ce que**
ladite au moins une autre caméra vidéo (3) est conçue pour capturer une lumière monochromatique ou **en ce qu'**au moins l'une desdites caméras vidéo (2, 3) est conçue sous forme de caméra à polarisation.

**5.** Ensemble de visualisation (1) selon la revendication 4,
**caractérisé en ce que**
au moins un dispositif à filtre de polarisation (22, 23) disposé en amont d'une caméra vidéo (2) destinée à capturer une lumière visible multicolore comprend un polariseur circulaire.

**6.** Ensemble de visualisation (1) selon l'une des revendications 1 à 5,
**caractérisé en ce que**
le dispositif d'évaluation (13) est conçu pour déterminer un ensemble de coefficients de matrice de Müller au moyen des images capturées par les caméras vidéo (2, 3) et pour déterminer un contraste de polarisation dérivé de celui-ci.

**7.** Ensemble de visualisation (1) selon l'une des revendications 1 à 6,
**caractérisé en ce que**

un dispositif à filtre de polarisation (20) disposé dans le trajet de faisceau (6) en aval du dispositif d'éclairage (4) et en amont d'une zone d'espace objet (5) est conçu pour polariser simultanément, de manière différente, au moins deux longueurs d'onde fixes s'écartant l'une de l'autre,
et/ou **en ce que**
au moins l'un des dispositifs à filtre de polarisation (20-24) comprend un filtre de longueur d'onde et/ou un polariseur sélectif en longueur d'onde.

**8.** Ensemble de visualisation (1) selon l'une des revendications 1 à 7,
**caractérisé en ce que**

le dispositif d'éclairage (4) est conçu pour émettre une lumière bichromatique
et/ou **en ce que**
le dispositif d'éclairage (4) est conçu pour émettre une lumière polychromatique et **en ce qu'**un dispositif à filtre de polarisation (20) disposé dans le trajet de faisceau (6) en aval du dispositif d'éclairage (4) et en amont d'une zone d'espace objet (5) comprend au moins un filtre coupe-bande et/ou plusieurs filtres passe-bande, et/ou **caractérisé par** au moins un séparateur de faisceau (12) qui est disposé dans le trajet de faisceau (6) entre une zone d'espace objet (5) et les caméras vidéo (2, 3).

**9.** Ensemble de visualisation (1) pour la microchirurgie, lequel comprend un dispositif d'imagerie (14), un dispositif d'éclairage (4) et un ensemble de détermination de polarisation,
**caractérisé en ce que**

l'ensemble de détermination de polarisation comprend deux caméras de polarisation (17), un trajet de faisceau partiel individuel étant associé à chacune des deux caméras de polarisation (17) dans le trajet de faisceau (6), et un dispositif d'évaluation (13),
dans lequel le dispositif d'évaluation (13) est conçu pour générer des images à contraste de polarisation au moyen des images capturées par les caméras de polarisation (17) et pour les afficher au moyen du dispositif d'imagerie (14), dans lequel
le dispositif d'évaluation (13) est conçu pour afficher, au moyen du dispositif d'imagerie (14), les images générées à contraste de polarisation de manière superposée à des images en lumière blanche qui sont capturées au moyen des deux caméras de polarisation (17).

**10.** Procédé pour générer une image à contraste de polarisation d'un objet (5) au moyen d'un ensemble de visualisation (1) pour la microchirurgie selon l'une des revendications 1 à 9,
**caractérisé en ce que**
le procédé comprend les étapes suivantes :

- projection d'ondes lumineuses (41) sur l'objet (5) au moyen du dispositif d'éclairage (4),
- capture d'ondes lumineuses (42), lesquelles sont rayonnées par l'objet (5) après une interaction de l'objet (5) avec les ondes lumineuses projetées, au moyen des au moins trois caméras vidéo (2, 3) ou des au moins deux caméras de polarisation (17),
- génération d'une image à contraste de polarisation (43) sur la base d'un état de polarisation généré des ondes lumineuses projetées et/ou d'un état de polarisation analysé des ondes lumineuses capturées.

**11.** Procédé selon la revendication 10,

**caractérisé en ce que**

des ondes lumineuses dans un état de polarisation défini sont projetées au moyen du dispositif d'éclairage (4) et d'un dispositif à filtre de polarisation (20) disposé dans le trajet de faisceau (6) en aval du dispositif d'éclairage (4) et en amont de l'objet (5),
et/ou **en ce que**
l'état de polarisation des ondes lumineuses reçues est analysé au moyen des au moins trois caméras vidéo (2, 3) et des dispositifs à filtre de polarisation (21-24) disposés en amont de celles-ci dans le trajet de faisceau (6) et/ou au moyen des caméras de polarisation (17).

12. Procédé selon l'une des revendications 10 à 11, **caractérisé en ce que**
une image à contraste de polarisation est générée au moyen du dispositif d'évaluation (13) sur la base d'un état de polarisation analysé des ondes lumineuses capturées, dans lequel un ensemble de coefficients de matrice de Müller est déterminé au moyen des images capturées par les caméras vidéo (2, 3) et/ou les caméras de polarisation (17), et un contraste de polarisation dérivé de celui-ci est déterminé.

13. Procédé selon l'une des revendications 10 à 12, **caractérisé en ce que**
l'image à contraste de polarisation est générée au cours d'un intervalle de temps inférieur à 50 ms et/ou **en ce que** la résolution de l'image stéréoscopique à contraste de polarisation générée s'écarte de moins de 10 % de la résolution d'une image correspondante sans contraste de polarisation.

14. Procédé selon la revendication 13,
**caractérisé en ce que**
l'image à contraste de polarisation est générée au cours d'un intervalle de temps inférieur à 50 ms et est affichée de manière superposée à une image multicolore.

15. Microscope, lequel comprend un ensemble de visualisation (1) selon l'une des revendications 1 à 9 ou est conçu pour la mise en œuvre d'un procédé selon l'une des revendications 10 à 14.

Fig. 1

Fig. 2

P0    P90    P45    P135    PC    PN

Fig. 3

Fig. 4

Fig. 5

Fig. 6

Fig. 7

Fig. 8

Fig. 9

Fig. 10

Fig. 11

Fig. 12

Fig. 13

Fig. 14

Fig. 15

**Fig. 16**

**Fig. 17**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2016091702 A1
- DE 102017100904 A1
- DE 10242983 A1
- CN 107490851 A
- DE 102018110806 A1
- WO 2016170816 A1

**In der Beschreibung aufgeführte Nicht-Patentliteratur**

- **JAEPYEONG CHA et al.** Real-time, label-free, intraoperative visualization of peripheral nerves and micro-vasculatures using multimodal optical imaging techniques. *Biomedical Optics Express*, 12 February 2018, vol. 9 (3), 1097-1110
- Physical Image Formation. **W. SINGER** ; **M. TOT-ZECK** ; **H. GROSS**. Handbook of Optical Systems, vol. 2, 475ff
- **REIHE**. Handbook of Optical Systems. Wiley VCH
- **H. ENGSTROM**. Coherency matrix polarization measurements: application to magnetooptic garnet films. *Appl. Opt.*, 1991, vol. 30, 1730-1734
- **A. PIGULA** ; **N. T. CLANCY** ; **S. ARYA** ; **G. B. HANNA** ; **D. S. ELSON**. Video-rate dual polarization multispectralendoscopic imaging. *International Society for Optics and Photonics*, 2015
- **WEI SHENG et al.** Quantitative Analysis of 4 × 4 Mueller Matrix Transformation Parameters for Biomedical Imaging. *Photonics*, 2019, vol. 6, 34
- **JAEPYEONG CHA et al.** Real-time, label-free, intraoperative visualization of peripheral nerves and micro-vasculatures using multimodal optical imaging techniques. *BIOMEDICAL OPTICS EXPRESS*, 12 February 2018, vol. 9 (3), 1097-1110
- **J. QI** ; **D. S. ELSON**. Mueller polarimetric imaging for surgical and diagnostic application. *J. Biophotonics*, 2017, vol. 10, 950-982
- **VIZET, J.** ; **REHBINDER, J.** ; **DEBY, S. et al.** In vivo imaging of uterine cervix with a Mueller polarimetric colposcope. *Sci Rep*, 2017, vol. 7, 2471, https://doi.org/10.1038/s41598-017-02645-9